# EUROPÄISCHE PATENTSCHRIFT

(11) **EP 3 455 629 B1**
(45) Veröffentlichungstag und Bekanntmachung des Hinweises auf die Patenterteilung: **28.07.2021**
(21) Anmeldenummer: 17723982.9
(22) Anmeldetag: 12.05.2017
(51) Int. Cl.: G01N 33/543, G01N 27/30

(54) **BIOSENSOR, VERFAHREN ZU SEINER HERSTELLUNG UND VERFAHREN ZUM NACHWEISEN EINES ANALYTEN MIT HILFE DES BIOSENSORS**
BIOSENSOR, METHOD FOR THE PRODUCTION THEREOF, AND METHOD FOR DETECTING AN ANALYTE USING THE BIOSENSOR
BIOCAPTEUR, PROCÉDÉ DE FABRICATION DE CELUI-CI ET PROCÉDÉ DE DÉTECTION D'UN ANALYTE À L'AIDE DU BIOCAPTEUR

(30) Priorität: 13.05.2016 DE 102016108979
(43) Veröffentlichungstag der Anmeldung: 20.03.2019
(73) Patentinhaber: Fraunhofer-Gesellschaft zur Förderung der angewandten Forschung e.V., 80686 München (DE)
(72) Erfinder: GRABBERT, Niels, 12489 Berlin (DE); MEYER, Vera, 10405 Berlin (DE); LANG, Klaus-Dieter, 13057 Berlin (DE); FIEDLER, Markus, 10967 Berlin (DE)
(74) Vertreter: Strehl Schübel-Hopf & Partner
(86) Internationale Anmeldenummer: PCT/EP2017/061479
(87) Internationale Veröffentlichungsnummer: WO 2017/194746

(56) Entgegenhaltungen:
- KR-A- 20160 004 421
- ZHANG XIAOYUE ET AL: "Ultrasensitive nonenzymatic immunosensor based on bimetallic gold-silver nanoclusters synthesized by simple mortar grinding r", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, Bd. 194, 2014, Seiten 64-70, XP028609189, ISSN: 0925-4005, DOI: 10.1016/J.SNB.2013.12.065
- DAN WU ET AL: "Simultaneous electrochemical detection of cervical cancer markers using reduced graphene oxide-tetraethylene pentamine as electrode materials and distinguishable redox probes as labels", BIOSENSORS AND BIOELECTRONICS, Bd. 54, 1. April 2014 (2014-04-01), Seiten 634-639, XP055389694, NL ISSN: 0956-5663, DOI: 10.1016/j.bios.2013.11.042
- KAI YANG ET AL: "Preparation and functionalization of graphene nanocomposites for biomedical applications", NATURE PROTOCOLS, Bd. 8, Nr. 12, 7. November 2013 (2013-11-07), Seiten 2392-2403, XP055295539, GB ISSN: 1754-2189, DOI: 10.1038/nprot.2013.146
- JANA VLACHOVA ET AL: "Utilization of graphene oxide electrophoretic deposition for construction of electrochemical sensors and biosensors", JOURNAL OF METALLOMICS AND NANOTECHNOLOGIES, Bd. 3, 1. Januar 2015 (2015-01-01), Seiten 57-63, XP055390412,
- KIM TRUC NGUYEN ET AL: "Graphene and Graphene Derivatives in Biosensing, Imaging, Therapeutics, and Genetic Engineering", REV. CELL BIOL. MOL. MEDICINE, Bd. 1, Nr. 1, 1. Januar 2015 (2015-01-01), Seiten 386-420, XP055389819,
- LONKAR SUNIL P ET AL: "Recent advances in chemical modifications of graphene", NANO RESEARCH, TSINGHUA UNIVERSITY PRESS, CN, Bd. 8, Nr. 4, 28. November 2014 (2014-11-28), Seiten 1039-1074, XP035497347, ISSN: 1998-0124, DOI: 10.1007/S12274-014-0622-9 [gefunden am 2014-11-28]
- CELIK NUMAN ET AL: "Graphene-based biosensors: methods, analysis and future perspectives", IET CIRCUITS DEVICES AND SYS, THE INSTITUTION OF ENGINEERING AND TECHNOLOGY, GB, Bd. 9, Nr. 6, 1. November 2015 (2015-11-01), Seiten 434-445, XP006054560, ISSN: 1751-858X, DOI: 10.1049/IET-CDS.2015.0235
- Anonymous: "Ethyleneamines", internet article, 1 January 2007 (2007-01-01), pages 1-76, XP055634720, Retrieved from the Internet: URL:http://www.huntsman.com/performance_pr oducts/Media%20Library/a_MC348531CFA3EA9A2 E040EBCD2B6B7B06/Products_MC348531D0B9FA9A 2E040EBCD2B6B7B06/Amines_MC348531D0BECA9A2 E040EBCD2B6B7B06/Ethyleneamines_MC348531D0 CD3A9A2E040EBCD2B6B7B06/files/ethyleneamin es_brochure_huntsman_ethyleneamines.pdf [retrieved on 2019-10-22]
- Anonymous: "Product Specification for Tetraethylenepentamine", internet article, 21 June 2010 (2010-06-21), page 1, XP055634724, Retrieved from the Internet: URL:https://www.sigmaaldrich.com/catalog/p roduct/aldrich/t11509?lang=en&region=NL [retrieved on 2019-10-22]
- Anonymous: "Tetraethylene pentamine | Sigma-Aldrich", internet article, 22 October 2019 (2019-10-22), pages 1-3, XP055634728, Retrieved from the Internet: URL:https://www.sigmaaldrich.com/catalog/p roduct/mm/814713?lang=en&region=NL [retrieved on 2019-10-22]

## Beschreibung

### Gebiet der Erfindung

Die vorliegende Erfindung betrifft einen Biosensor, ein Verfahren zur Herstellung desselben, einen den Biosensor tragenden Chip sowie ein Verfahren zum Nachweisen eines zu detektierenden Analyten (eines Antigens oder Biomarkers).

### Stand der Technik

Graphen ist eine ultradünne Schicht aus zu einem Bienenwabenmuster verbundenen Kohlenstoffatomen. Es bietet aufgrund seiner hervorragenden elektrischen Fähigkeit in Kombination mit einer hohen chemischen und mechanischen Stabilität weitreichende Anwendungsmöglichkeiten. Mittels chemischer Funktionalisierung gelingt es nicht nur, stabile Graphensuspensionen herzustellen, sondern auch seine Wechselwirkung mit anderen Materialien zu kontrollieren, was von großer Bedeutung für die Realisierung von Produkten ist. Für diese Zwecke wird vor allem reduziertes Graphenoxid (rGO) verwendet (Wojtoniszak, M. et al. (2012), "Synthesis, dispersion, and cytocompatibility of graphene oxide and reduced graphene oxide", Colloids and Surfaces B: Biointerfaces Volume 89, pages 1-294).

Beispielsweise wird modifiziertes Graphen über selektive chemische Wechselwirkungen als chemischer oder biochemischer Sensor für Analytmoleküle eingesetzt. Aus dem Stand der Technik ist mit Polyethylenglykol (PEG) funktionalisiertes rGO bekannt. Weiterhin kann rGO-PEG-NH₂ ausgenutzt werden, um während eines Immunoassays Bakterien abzutöten. Damit kann die Detektionsgenauigkeit erhöht und unerwünschtes Ergebnisrauschen, durch z.B. Bakterienwachstum, verhindert werden ("Highly Sensitive Detectionof DNA Hybridization on CommercializedGraphene-Coated Surface Plasmon Resonance Interfaces", Anal. Chem., 2014, 86 (22), pp 11211-11216).

Andererseits ist aus dem Stand der Technik die kovalente Modifizierung von Antikörpern oder Fragmenten davon mit PEG ("PEGylation") bekannt, mit der Antikörper oder deren Fragmente stabilisiert werden können ("PEGylation of antibody fragments for half-life extension", Methods Mol. Biol. 2012; 901:233-46). Zudem wird PEG als Abstandshalter für Antikörper in einem Analysemedium eingesetzt, um die Reaktionskinetik zu verbessern.

Funktionalisiertes Graphen wurde auch bereits für die simultane Detektion von Adenin und Guanin mittels Voltammetrie in DNA vorgeschlagen, siehe Ke-Jing Huang et al. in Colloids and Surfaces B: Biointerfaces 82 (2011) 543-549.

Aufgabe der vorliegenden Erfindung ist es, einen Biosensor bereitzustellen, mit dem ein Analytmolekül mit besonders hoher Sensitivität mit einer elektrischen Detektionsmethode, ausgewählt unter Impedanzspektrometrie und zyklischer Voltammetrie, innerhalb einer extrem kurzen Detektionszeit detektiert werden kann.

ZHANG X ET AL: "Ultrasensitive nonenzymatic immunosensor based on bimetallic gold-silver nanoclusters synthesized by simple mortar grindung", SENSORS AND ACTUATORS B, vol. 194, 2014, Seiten 64-70; WU D ET AL: "Simultaneous electrochemical detection of cervical cancer markers using reduced graphene oxide-tetraethylene pentamine as electrode materials and distinguishable redox probes as labels", BIOSENSORS AND BIOELECTRONICS, vol. 54, 2014, Seiten 634-639; und KR 2016 0004421 A offenbaren Biosensoren, die eine Sensorbasis mit einem isolierenden Substrat und mindestens einer darauf befindlichen, elektrisch leitenden Arbeitselektrode, auf mindestens eine der Arbeitselektroden aufgebrachtes reduziertes Graphenoxid, an das reduzierte Graphenoxid kovalent gebundene Abstandshalter unterschiedlicher Länge sowie ein an die Abstandshalter kovalent gebundenes Fängermolekül enthalten.

### Zusammenfassende Darstellung der Erfindung

Die Aufgabe wird durch Bereitstellen eines Biosensors mit mindestens einer mit reduziertem Graphenoxid belegten Arbeitselektrode gelöst, wobei ein Antikörperfragment Fab bzw. ein anderes Fängermolekül kovalent über einen Abstandshalter an das reduzierte Graphenoxid gebunden ist.

Die vorliegende Erfindung ist in den Patentansprüchen definiert.

Der erfindungsgemäße Biosensor umfasst folgende Komponenten:
(a) eine Sensorbasis mit einem isolierenden Substrat und mindestens einer darauf befindlichen, elektrisch leitenden Arbeitselektrode,
(b) auf mindestens eine der Arbeitselektroden aufgebrachtes reduziertes Graphenoxid (rGO) in der Form von flächigen Flocken einer Größe, das heißt maximalen Länge, von 0,2 bis 3,0 µm, wobei die Flächen von mindestens 50 % der Graphenoxidflocken von der Ebene der Arbeitselektrodenfläche, auf der sie aufgetragen sind, abweichen,
(c) an das reduzierte Graphenoxid kovalent gebundene Abstandshalter ("Spacer") unterschiedlicher Länge,
(d) jeweils ein an die Abstandshalter kovalent gebundenes Fängermolekül.

Bevorzugt ist ein erfindungsgemäßer Biosensor, wobei das Fängermolekül ein Antikörperfragment Fab oder ein L-Aptamer ist.

Bevorzugt ist ein erfindungsgemäßer Biosensor, bei dem die Komponenten (b) bis (d) auf eine Arbeitselektrode, wobei der Biosensor weiterhin eine Referenzelektrode aufweist, oder auf mit Wechselstrom beaufschlagbare Interdigitalelektroden aufgebracht sind.

Bevorzugt ist ein erfindungsgemäßer Biosensor, wobei als Abstandshalter endseitig aminierte Polyalkylenglycol-Moleküle unterschiedlicher Länge und/oder Polyalkylenpolyamin-Moleküle unterschiedlicher Länge dienen.

Der Abstandshalter weist vorzugsweise eine optional durch Sauerstoffatome und/oder Aminogruppen unterbrochene Kohlenwasserstoffkette mit einer Kettenlänge von vorzugsweise von 5 bis 10.000 Atomen auf. Vorzugsweise ist dieser Abstandshalter über Aminogruppen an das rGO angebunden; alternativ sind andere Kupplungsgruppen möglich, die durch eine Anbindung geeigneter reaktiver Gruppen am Graphenoxid vor dessen Reduktion vorhandene freie Carbonsäuregruppen oder dergleichen erhalten werden können. Insbesondere wird der Abstandshalter durch Umsetzen mindestens difunktioneller Amine gegebenenfalls unterschiedlicher Länge wie Polyethylenglycol-NH₂ und/oder eines Polyalkylenamins wie z.B. Tetraethylenpentamin mit Graphenoxid und, nach Reduktion des Graphenoxids, Umsetzen mit dem Antikörperfragment Fab bzw. mit einem anderen Fängermolekül erhalten.

In diesem Zusammenhang sollen die Ausdrücke "von Polyalkylenglycol-NH₂ abgeleitet", "von Polyethylenglycol-NH₂ abgeleitet", "von Polyalkylenamin abgeleitet" und "von Tetraethylenpentamin abgeleitet" bedeuten, dass ein endständig mit NH₂-Gruppen modifiziertes Polyalkylenglycol/Polyethylenglycol bzw. ein Oligoalkylenamin/ Tetraethylenpentamin mit Graphen und mit dem Antikörperfragment Fab bzw. mit einem anderen Fängermolekül umgesetzt wurde, so dass der entstehende Abstandshalter mindestens zwei Kopplungsgruppen aufweist, wobei über eine dieser Gruppen das reduzierte Graphenoxid und über die andere dieser Gruppen das Antikörperfragment Fab bzw. ein anderes Fängermolekül gebunden ist. Die Bindung ist in der Regel jeweils als -C(O)NH-Kopplungsgruppe ausgestaltet.

Der Abstandshalter umfasst vorzugsweise sowohl Abstandshalter, die von Polyethylenglycol-NH₂ unterschiedlicher Länge abgeleitet sind, als auch Abstandshalter, die von Tetraethylenpentamin abgeleitet sind.

Die Anbindung des Antikörperfragments bzw. eines anderen Fängermoleküls an den Abstandshalter erfolgt vorzugsweise durch Umsetzen einer Carboxylgruppe des Antikörperfragments Fab bzw. eines anderen Fängermoleküls mit einer Aminogruppe des Abstandshalters. Durch diese Umsetzung entstehen Amidgruppen.

Vorzugsweise ist also der Abstandshalter über Amidgruppen auch an das reduzierte Graphenoxid, also an das Graphen, gebunden. Diese Umsetzung kann z.B. mittels eines im Stand der Technik bekannten Verfahrens unter Einsatz einer Carbodiimidverbindung als Katalysator erfolgen.

In einer Ausführungsform sind eventuell vorhandene freie funktionelle Gruppen des reduzierten Graphenoxids und/oder des Abstandshalters blockiert.

Diese Blockierung ist erhältlich durch Umsetzen mit funktionelle Gruppen enthaltenden Verbindungen, beispielsweise mit Proteinen. Eine übliche Zusammensetzung, ein sogenannter Blocking-Buffer, zur Blockierung von freien funktionellen Gruppen ist Milchpulver/1:10PBS/0,1% Tween20.

Der fertig vorbereitete Biosensor kann über eine Stabilisierungsschicht geschützt werden. Diese kann beispielsweise aus Zucker bestehen. Wird der Biosensor zum Messen eingesetzt, löst sich der Zucker in den Mess- und Spülflüssigkeiten auf.

Bevorzugt ist ein erfindungsgemäßer Biosensor, worin die kovalente Bindung zwischen dem Abstandshalter und dem Fängermolekül durch Umsetzen einer Carboxylgruppe des Antikörperfragments Fab als Fängermolekül mit einer Aminogruppe des Abstandshalters oder durch Umsetzen einer mittels Azidoacetylchlorid azidierten Aminogruppe mit einem DNA- oder RNA-Molekül als Fängermolekül erhältlich ist.

Ein erfindungsgemäßes Verfahren zum Herstellen eines Biosensors umfasst die folgenden Schritte:
(i) das Bereitstellen einer Sensorbasis mit einem isolierenden Substrat und mindestens einer darauf befindlichen, elektrisch leitenden Arbeitselektrode,
(ii) das Bereitstellen von reduziertem Graphenoxid, das mit difunktionellen Aminoverbindungen unterschiedlicher Länge funktionalisiert wurde, in der Form von flächigen Flocken einer Größe, das heißt maximalen Länge, von 0,2 bis 3,0 µm,
(iii) das Auftragen des in Schritt (ii) bereitgestellten funktionalisierten reduzierten Graphenoxids auf die mindestens eine Arbeitselektrode sowie
(iv) das Umsetzen eines Fängermoleküls mit dem in Schritt (iii) erhaltenen Produkt,
wobei das reduzierte Graphenoxid zwischen den Schritten (ii) und (iii) in einem Lösungsmittel unter Ultraschallbehandlung dispergiert wird, und wobei die Flächen von mindestens 50 % der Graphenoxidflocken von der Ebene der Arbeitselektrodenfläche, auf der sie aufgetragen sind, abweichen.

Bevorzugt ist ein erfindungsgemäßes Verfahren, wobei ein erfindungsgemäßer Biosensor erhalten wird.

Ein erfindungsgemäßes Verfahren zum Herstellen eines Biosensors umfasst die folgenden Schritte:
(i) das Bereitstellen einer Sensorbasis mit einem isolierenden Substrat und mindestens einer darauf befindlichen, elektrisch leitenden Arbeitselektrode,
(ii) das Bereitstellen von reduziertem Graphenoxid in der Form von flächigen Flocken einer Größe, das heißt maximalen Länge, von 0,2 bis 3,0 µm,
(iii) das Funktionalisieren des reduzierten Graphenoxids mit difunktionellen Aminoverbindungen,
(iv) das Auftragen des bereitgestellten funktionalisierten reduzierten Graphens auf die mindestens eine Arbeitselektrode mittels elektrophoretischer Abscheidung und
(v) das Umsetzen eines Fängermoleküls mit dem in Schritt (iv) erhaltenen Produkt,
wobei das reduzierte Graphenoxid zwischen den Schritten (ii) und (iii) oder zwischen den Schritten (iii) und (iv) in einem Lösungsmittel unter Ultraschallbehandlung dispergiert wird, und wobei die Flächen von mindestens 50 % der Graphenoxidflocken von der Ebene der Arbeitselektrodenfläche, auf der sie aufgetragen sind, abweichen.

In den erfindungsgemäßen Verfahren kann anstelle der Bereitstellung von mit Abstandshalter modifiziertem, reduziertem Graphenoxid und dessen Aufbringen auf die Arbeitselektrode zunächst Graphenoxid auf die Arbeitselektrode aufgetragen und anschließend das Graphenoxid mit dem Aminogruppen tragenden Vorläufer für den Abstandhalter, z.B. mit Polyethylenglycol-NH₂ und/oder Tetraethylenpentamin, modifiziert werden, worauf das Graphenoxid (zu rGO) reduziert wird.

### Vorteile der Erfindung

Das erfindungsgemäß genutzte Graphen liegt als rGO (reduziertes Graphenoxid) vor und hat somit gewünscht einzigartige elektrische Eigenschaften, vor allem eine extrem hohe Ladungsträgerbeweglichkeit. Diese ist ausschlaggebend für den in der vorliegenden Erfindung nutzbaren Effekt. Bei Wechselwirkung mit anderer Materie in der Nähe des rGO wird diese Beweglichkeit nämlich gestört. Dadurch kommt es schon bei kleinsten Mengen von interagierender Materie zu einer signifikanten Veränderung der elektrischen Materialeigenschaften, beispielsweise des Widerstandes. Daraus ergibt sich die herausragende Verwendbarkeit von Graphen als Sensormaterial für die vorliegenden Zwecke. Reduziertes Graphenoxid ist überdies kostengünstig in der Herstellung und einfach auf gewünschte Zielstrukturen abscheidbar.

Durch das Vorhandensein eines Oberflächenabstandhalters variabler Länge wie voranstehend beschrieben, insbesondere unterschiedlicher Länge, wird die Fab-Mobilität erhöht und dadurch die Reaktionskinetik mit den Antigenen im Analysemedium verbessert.

Der Abstandshalter besitzt vorzugsweise endseitig jeweils Aminogruppen, Eine dieser Gruppen wird zur Anbindung an das rGO genutzt, die andere dient zur kovalenten Bindung an das Fab. Damit wird es möglich, Fab auf günstige und gängige Weise anzubinden. Weiterhin ermöglicht diese kovalente Bindung eine mehrfache Nutzung des Sensors. Fab-Antigen-Bindungen können nach gewünschter Messung aufgebrochen und der Biosensor erneut genutzt werden. Dadurch wird ein gewisser Recycling-Effekt des Sensors realisiert.

Mit PEG funktionalisiertes Graphen ist bio-kompatibel und kann daher leichter als reines rGO aus einem menschlichen oder tierischen Körper entfernt werden. Dies stellt einen wichtigen Punkt für die Arbeitssicherheit dar und reduziert außerdem die vom Produkt ausgehende Umweltbelastung.

Durch die Verwendung von Abstandshaltern unterschiedlicher Länge kann die Anzahl der Fab-Fragmente pro Flächeneinheit oder Volumeneinheit deutlich erhöht werden, so dass die Leistungsfähigkeit des Biosensors verbessert werden kann.

Die Verwendung von Fab-Fragmenten hat mehrere Vorteile. Sie können monovalent an passende Antigene binden. Außerdem werden unspezifische Bindungen des Antikörper-Fc-Anteils komplett eliminiert, da dieser Abschnitt bei Nutzung eines Fab-Fragments nicht mehr vorhanden ist. Die Stabilität von Fab-Fragmenten, die geringer ist als die von ganzen Antikörpern, wird durch die Bindung an PEG erhöht. Außerdem können mehr Fab-Fragmente auf der Elektrode untergebracht werden als es bei Verwendung von ganzen Antikörpern der Fall ist.

Die dreidimensionale Strukturanordnung des Graphens durch homogene Dispergierung von Graphenflocken und anschließende elektrophoretische Abscheidung der strukturierten Graphenflocken hat den Vorteil, dass die Oberfläche des Graphens vergrößert wird und somit pro Flächeneinheit des Biosensors mehr Marker und folglich eine höhere Empfindlichkeit des Biosensors ermöglicht wird.

### Beschreibung der Figuren

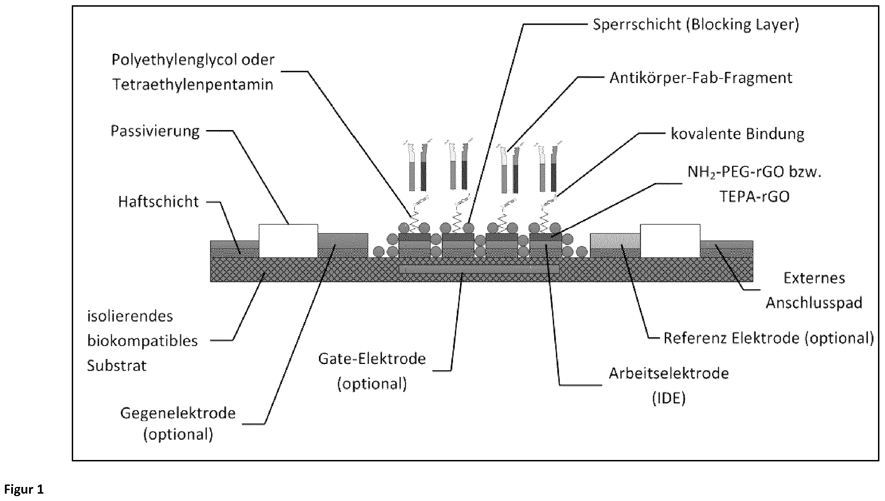

Figur 1 zeigt in einer schematischen Darstellung den Grundaufbau des erfindungsgemäßen Biosensors bzw. Chips mit einem isolierenden, biokompatiblen Substrat, einer Haftschicht für die Elektroden, erfindungsgemäß belegten Interdigitalelektroden, einer (grundsätzlich optionalen) Gegenelektrode, einer (grundsätzlich optionalen) Referenzelektrode, einer (grundsätzlich optionalen) Gateelektrode im isolierenden Substrat, einer Passivierung (z.B. in Form eines Rings) sowie einem externen Anschlusspad. Die kovalente Anbindung des Fab-Fragments ist, wie auch in allen anderen nachfolgenden Figuren, nicht als -N=C=N- sondern als -C(O)NH-Bindung zu lesen.
Figur 2a zeigt eine schematische Darstellung bei Einfluss einer Analyseflüssigkeit.
Figur 2b zeigt eine schematische Darstellung der selektiven Biomarkerbindung unter der Analyseflüssigkeit.
Figur 3 zeigt eine schematische Darstellung unterschiedlicher Messmethoden.
Figur 4 zeigt ein schematischesSchaltbild bei Nutzung einer Ag/AgCl₂-Referenzelektrode auf Chip-Ebene.
Figur 5 zeigt eine schematische Darstellung einer Multiplex-Detektion mittels Verwendung von vier unterschiedlichen Fab-Fragmenten auf vier IDE-Strukturen, einer Arbeitselektrode pro IDE-Struktur und einer Referenzelektrode auf einem einzigen Biochip.
Figur 6 zeigt einen schematischen Entwurf eines Graphen-Biosensor-FETs, welcher über ein Substrat-Gate oder über Flüssig-Gate (Detektionsflüssigkeit leitend) gesteuert werden kann.
Figur 7 zeigt die schematische Darstellung einer IDE-Fingerstruktur.
Figur 8 zeigt ein vereinfachtes elektrisches Ersatzschaltbild eines IDE-Fingers, ohne und mit Analyt ("Biomarker"-)Bindung in Elektrolytlösung.
Figur 9 zeigt die schematische Veränderung der Impedanz in Abhängigkeit der Anwesenheit eines Biomarkers sowie bei verschiedenen Kreisfrequenzen ω, dargestellt in der Komplexenzahlenebene.
Figur 10 zeigt die schematische Darstellung der Messung unter Einsatz eines Operationsverstärkers (OPV) und einer Gegenelektrode.
Figur 11 zeigt die schematische Darstellung der Einstellung des Arbeitspunktes des Graphens über U_{Gate}. Als Drain und Source können hier die IDEs eingesetzt werden; alternativ werden die IDEs auf ein Potential gelegt und gegen die Gegen- oder Arbeitselektrode vermessen. Zwischen Drain und Source wird sodann entweder der Stromfluss oder die Spannung gemessen.

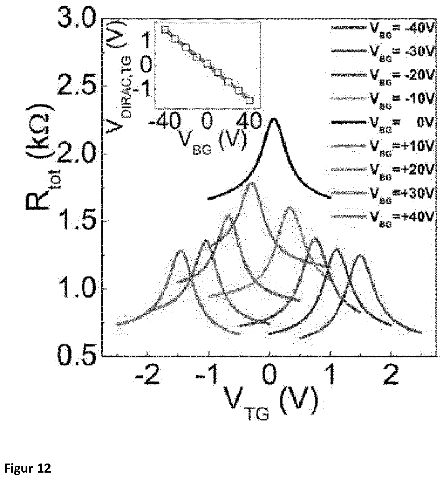

Figur 12 zeigt die Widerstandsänderung (Arbeitspunktverschiebung) bei verschiedenen Gate-Spannungen (VTG). Die Widerstandsänderung wird aus der IV-Messung (Messung des Stromflusses oder der Spannung) ermittelt.
Figur 13 zeigt eine REM-Aufnahme einer IDE mit abgeschiedenem Graphen.
Figur 14 zeigt die schematische Darstellung der Chemolumineszenzdetektion mittels Sandwich-ELISA.
Figur 15 zeigt ein Impedanzspektrum mit und ohne GST als Analyten (Biomarker). Die Detektion von 10 ng/ml GST im Messelektrolyt konnte gezeigt werden. Hierbei ergab sich eine Differenz der Impedanz ohne und mit Biomarker um signifikante 1000 Ohm.
Figur 16 zeigt die Reaktionskinetik des Biosensors mit und ohne 10 ng/ml GST im Messelektrolyten. Es konnte eine Detektionszeit von etwa 200 s ermittelt werden.
Figur 17 zeigt schematisch die Erhöhung der Oberfläche durch Erzeugung von dreidimensionalen Strukturen aus zweidimensionalen rGO-Flocken.
Figur 18 zeigt ein REM-Bild von dreidimensional angeordneten Graphenflocken.
Figur 19 ist eine schematische Darstellung der Funktionalisierung mittels Klick-Chemie und Amidbindung.

### Detaillierte Beschreibung der Erfindung

Der in der vorliegenden Erfindung benutzte Ausdruck "Fängermolekül" bezeichnet ganz allgemein eine Substanz, die eine andere Substanz nichtkovalent binden kann. Diese Bindung ist vorzugsweise selektiv. Beispiele von Fängermolekülen sind Antikörper, insbesondere Fab-Fragmente, und Aptamere, insbesondere aus DNA und/oder RNA. L-Aptamere sind bevorzugt. Weitere denkbare Wechselwirkungen unter Einsatz einer Komponente als Fängermolekül sind Rezeptor-Ligand-Wechselwirkungen oder speziell die Biotin-Streptavidin-Wechselwirkung. Die in dieser Anmeldung genannten Fängermoleküle können aus einer Molekülspecies bestehen, also aus Proteinen, insbesondere Fab-Fragmenten, oder Aptameren. Die Fängermoleküle können jedoch auch ein Gemisch verschiedener solcher Molekülspecies bezeichnen. Die in der vorliegenden Beschreibung auf Antikörperfragmente Fab bezogenen Erläuterungen treffen entsprechend auch auf andere Fängermoleküle zu.

Die hier verwendeten Bezeichnungen "Analyt" oder "Biomarker" umfassen jede Verbindung oder Substanz, die ein Antigen eines Antikörpers bzw. ein Substrat eines anderen Fängermoleküls sein kann. Die Erfindung kann beispielsweise eingesetzt werden zur Bestimmung von Pathogenen, Stoffwechselprodukten und anderen medizinisch wichtigen Biomarkern im Blut, Speichel, in rektalen Ausscheidungen und anderen Körperflüssigkeiten bei Mensch und Tier; zur Überwachung von Trinkwasser und Abwässern; zur Qualitätskontrolle in der Lebensmittelindustrie; oder in der Pharmazie und Arzneimittelentwicklung oder in der Sicherheitstechnik zur Detektion von chemischen oder biologischen Gefahrstoffen.

Der in der vorliegenden Erfindung verwendete Ausdruck "rGO" bezeichnet das durch Oxidation und anschließende Reduktion von Graphen erhältliche Produkt, also reduziertes Graphenoxid. Die Ausdrücke "reduziertes Graphenoxid" und "reduziertes Graphen" werden hier synonym verwendet.

Der in der vorliegenden Erfindung verwendete Ausdruck "Antikörperfragment Fab" bezeichnet das Fab-Fragment eines Antikörpers (Immunglobulins), beispielsweise IgG. Ein hier synonym verwendeter Ausdruck ist "Fab-Fragment" oder kurz "Fab". Der Ausdruck "Fab" wird sowohl für einzelne Moleküle als auch für die Gattung verwendet, wie es auch bei der Bezeichnung von chemischen Verbindungen üblich ist.

Bei dem erfindungsgemäßen Biosensor handelt es sich bevorzugt um einen On-Chip-Biosensor, welcher äußerst geringe Konzentrationen von definierten Biomarkern in Flüssigkeiten detektieren kann.

Als Sensor-Basis dient ein Substrat mit biokompatibler Oberfläche, beispielsweise aus Glas, biokompatiblen Polymeren, Si₃N₄, verschiedenen Siliziumoxiden, z.B. amorphem oder aus epitaktischem SiO₂ oder anderen geeigneten Materialen.

Das Substrat selbst kann isolierend, ein Halbleiter wie Silizium oder auch elektrisch leitend sein, sofern es in geeigneter Weise auf seiner Oberfläche isoliert ist.

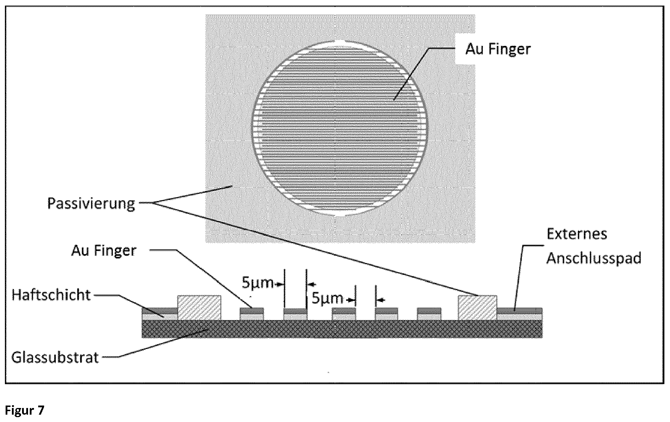

Der erfindungsgemäße Biosensor besitzt eine Basis mit einem isolierenden Substrat, auf das mindestens eine Arbeitselektrode aufgebracht ist. Bei jeder dieser Arbeitselektroden kann es sich um eine einzelne Flächenelektrode oder um Interdigitalelektroden (IDEs) handeln (Figur 7). Weitere Elektroden sind optional. Werden Interdigitalelektroden als positive und negative Elektrode geschaltet, kann beispielsweise eine weitere Elektrode als Referenzelektrode eingesetzt werden. Wird nur eine Arbeitselektrode eingesetzt, wird sie gegen eine Gegenelektrode geschaltet; eine Referenzelektrode kann auch in dieser Variante eingesetzt werden, um die Arbeitselektrode oder die Gegenelektrode zu überwachen. In einer besonderen Ausführungsform, die mit allen voranstehenden Ausgestaltungen kombinierbar ist, wird eine (Substrat-)Gate-Elektrode eingesetzt. Diese kann über typische, dem Fachmann bekannte Halbleiterprozesse wie Dünnschichttechniken, Dickschichttechniken oder Ionenimplantation hergestellt werden. Die Referenzelektrode kann eine als Paste auf die Oberfläche des Sensors gedruckte Ag/AgCl-Elektrode sein. Werden Interdigitalelektroden eingesetzt, können, müssen aber nicht, beide Elektrodenstrukturen vollständig mit dem modifizierten rGO beschichtet sein, auch wenn nur eine davon als Arbeitselektrode und die andere z.B. als Pseudo-Referenzelektrode dienen soll, wie weiter unten beschrieben.

Auf das Substrat kann zuerst eine strukturierte Haftschicht aufgebracht werden, auf der die metallischen Elektrodenstrukturen besonders gut haften, beispielsweise eine Ti-Schicht. Auf dieser wird dann das Metall der Arbeitselektrode(n), beispielsweise Aluminium, Platin, Gold oder ein anderes Edelmetall oder ein sonstiges elektrisch leitendes Material, das der Fachmann kennt, aufgebracht. Die strukturierte Ebene der Elektroden kann gleichermaßen auch für externe Anschlusspads eines Chips genutzt werden, wie z.B. aus den Figuren 1 bis 4 ersichtlich. Eine Passivierungsschicht, beispielsweise aus einem Kunststoffmaterial wie SU8, kann z.B. rahmenförmig um die Elektroden angeordnet sein, um ggf. offenliegende elektrische Leiterbahnen gegenüber der Messflüssigkeit zu schützen und gleichzeitig für diese eine Vertiefung zu bilden.

Alle Elektroden können auf Chip-Ebene integriert sein.

Figur 7 zeigt die schematische Darstellung einer IDE-Fingerstruktur (Interdigitalstruktur).

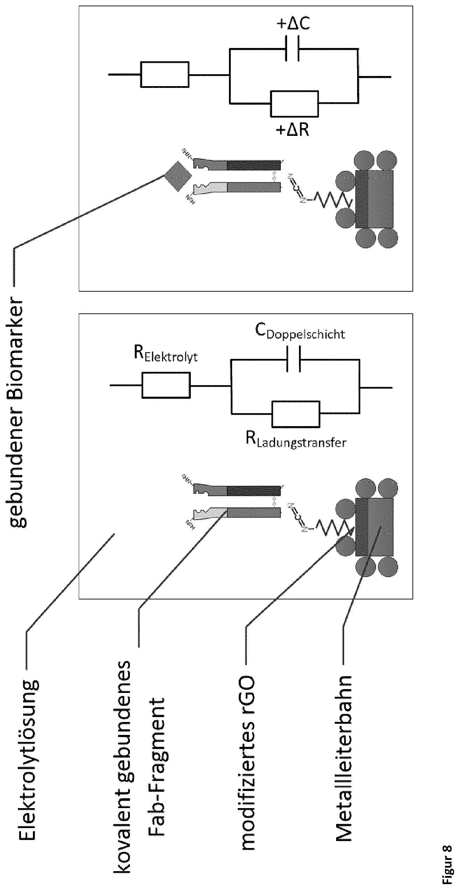

Figur 8 zeigt ein vereinfachtes elektrisches Ersatzschaltbild eines IDE-Fingers, ohne und mit Biomarkerbindung in Elektrolytlösung.

Prinzipiell sollten die Abstände der IDE-Fingerstruktur <5µm sein (Figur 7). Es ist bekannt, dass mit verringerten Fingerabständen die Sensorsensitivität exponentiell ansteigt.

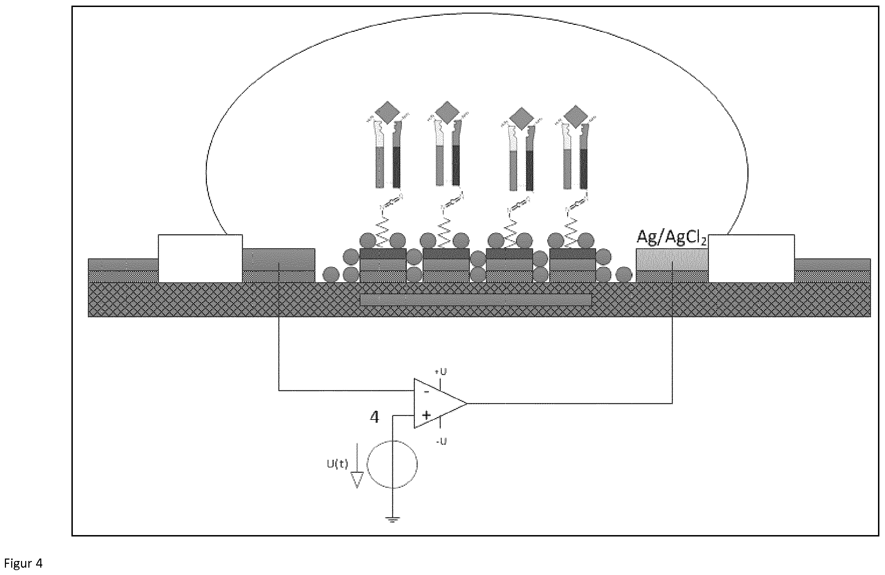

Die miniaturisierte Ag/AgCl₂-Referenzelektrode kann auf Chip-Ebene integriert werden (Figur 4); sie wird wie erwähnt vorzugsweise aufgedruckt.

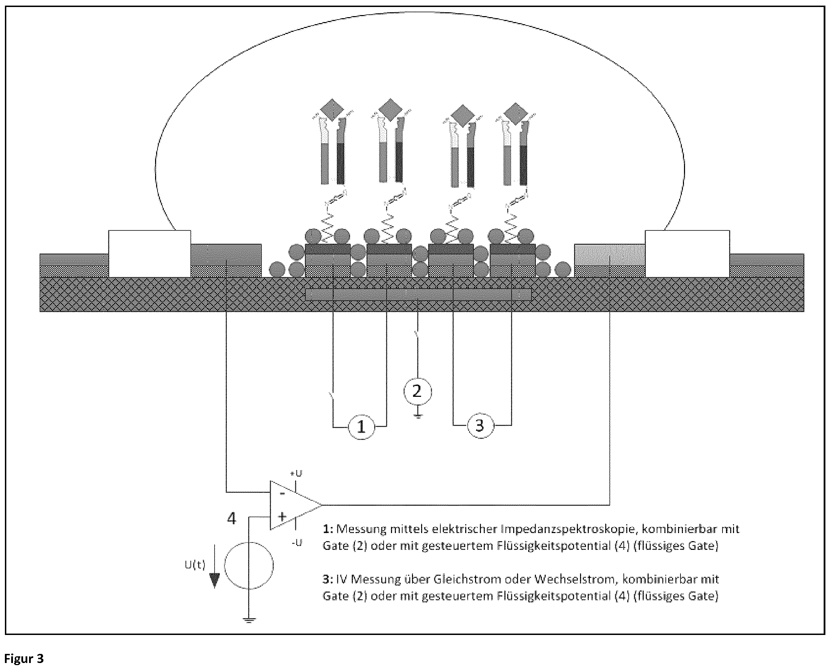

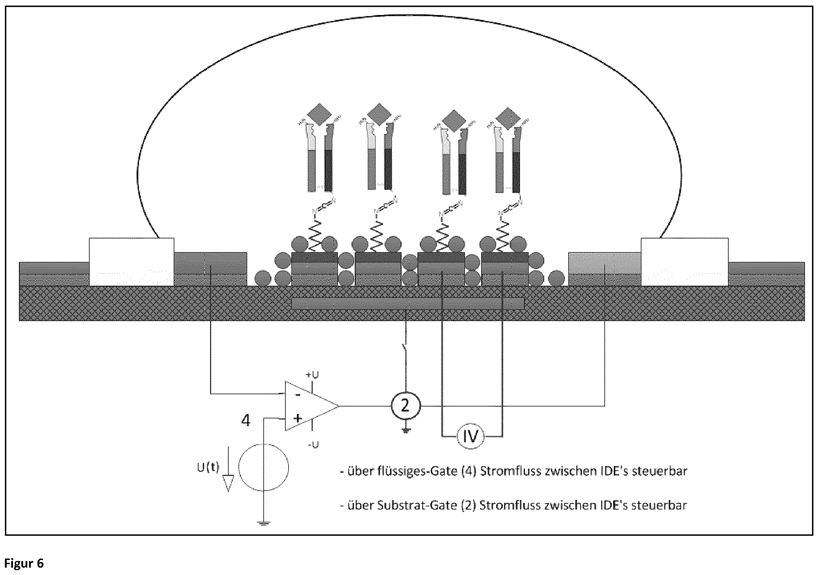

Der erfindungsgemäße Biosensor kann unter Nutzung von Gate-Elektroden (Substrat-Gate und/oder Flüssig-Gate; Figuren 3 und 6) als FET (Feldeffekt-Transistor) aufgebaut sein, was zu höherer Sensitivität und zur Möglichkeit der Signaladaption führt. Die Substrat-Gate-Elektrode befindet sich dabei isoliert im Träger (ihre elektrische Ansteuerung ist in den Figuren nicht gezeigt); wird sie über ein elektrisches Potential aktiviert, beeinflusst sie die elektronische Struktur des rGO. Unter "Flüssig-Gate" ist zu verstehen, dass mittels Gegenelektrode und Referenzelektrode im Elektrolyten ein definiertes elektrisches Potential eingestellt werden kann; der Stromfluss zwischen den IDEs kann damit gesteuert werden.

In dem erfindungsgemäßen Biosensor bildet vorzugsweise ein Aufbau mit einer Interdigitalelektrode (IDE) als Arbeitselektrode auf einem Substrat die Sensorbasis (Figur 1).

Auf der Arbeitselektrode bzw. den Interdigitalelektroden befindet sich Graphen. Vorzugsweise ist/sind die gesamte Elektrodenoberflächen(n) der Arbeitselektrode(n) damit belegt.

### Herstellung von reduziertem Graphen(oxid)

Grundsätzlich wird Graphen mittels zweier Hauptmethoden hergestellt. Zum einem mittels CVD (Chemical Vapor Deposition), wobei eine Graphenschicht aus der Gasphase (z. B. aus CH₄) katalytisch auf z.B. Kupfer oder Nickel abgeschieden wird. Diese Form der Herstellung ist sehr kostenintensiv, da unter anderem hier meist ein Transferschritt benötigt wird, welcher das katalytisch abgeschiedene Graphen auf die eigentliche Wunschstruktur überträgt. Das auf diese Weise erzeugbare Graphen ist jedoch sehr defektfrei und hat perfekte elektrische Zieleigenschaften. Nachteilig ist jedoch, dass es schwierig ist, funktionelle Gruppen an dieses Graphen anzulagern, weil kaum Strukturdefekte als Verbindungsglied für solche Gruppen vorhanden sind. Man kann künstlich wiederum Strukturdefekte einprägen (z.B. durch eine Plasmabehandlung), um dann gewünschte funktionelle Gruppe an die Graphenoberfläche zu binden. Dabei verschlechtern sich jedoch die elektrischen Eigenschaften, welche diese Herstellungsvariante gerade besonders macht.

Nach der anderen Methode wird Graphen durch chemisches und/oder mechanisches Exfolieren von Graphit hergestellt. Der große Vorteil gegenüber dem CVD-Graphen ist die vergleichsweise kostengünstige Herstellung sowie die mögliche Massenproduktion. Graphit lässt sich vereinfacht als eine Säule aus gestapelten Graphenschichten auffassen. Meistens wird für die Exfolierung des Graphits die modifizierte HUMMERS-Methode verwendet, wobei der Graphit mittels chemischer Lösung stark oxidiert wird. Dadurch entstehen Hydroxyl- und Epoxygruppen in der Zwischenschicht des Graphits, weswegen sich die einzelnen Schichten weiter voneinander entfernen und der Schichtaufbau an Stabilität verliert. Weiterhin entstehen an den Schichträndern Hydroxyl-, Carbonyl- und Carboxylgruppen. Die Oberfläche ist somit stark hydrophil. Bei Zugabe eines starken polaren Lösemittels siedelt dieses sich um die einzelnen funktionellen Oberflächengruppen an. Dabei exfolieren die einzelnen Schichten und es entsteht eine Suspension aus GO (Graphenoxid). Das Exfolieren kann noch durch Einprägung von Ultraschall optimiert werden. Die Strukturdefekte können nun ausgenutzt werden, um definierte funktionelle Gruppe an die Oberfläche zu binden. Vorzugsweise in diesem Stadium wird das Graphen mit dem Abstandshalter ("Spacer") modifiziert, insbesondere durch Anbindung einer aminogruppenhaltigen Verbindung auf der Graphenoberfläche befindliche COOH-Gruppen. Da jedoch auch nach dem "Verbrauch" eines Teils der COOH-Gruppen durch die hohe Defektanzahl die elektrischen Eigenschaften des Graphens eher moderat ausfallen, kann eine anschließende chemische oder thermische Reduktion der durch die Oxidation entstandenen Gruppen zur Rückführung eines fast perfekten Gitters ausgenutzt werden, wie es vorliegend der Fall ist. Damit ändert sich die Materialbezeichnung klassisch in rGO (für reduziertes Graphenoxid), und rGO nimmt ähnlich gute elektrische Eigenschaften wie CVD-Graphen an.

Durch die chemische und mechanische Exfolierung wird das reduzierte Graphen typischerweise deformiert und auch gefaltet.

Weiterhin wird CVD-Graphen wie erwähnt auf einer Arbeitsoberfläche abgeschieden und kann nur durch gewisse Transferschritte auf eine Zielstruktur übertragen werden. Das rGO hingegen liegt nach der Herstellung oft als "Pulver" vor, das aus rGO-Flakes besteht, welche in Suspension gebracht werden können, um sie dann direkt auf die gewünschte Struktur aufzubringen.

Typischerweise wird rGO mit dem oben beschriebenen Verfahren hergestellt. Unterschiedliche Funktionalisierungsschritte während der Herstellung führen hierbei zur veränderten Oberflächenstrukturen, die dann gezielt genutzt werden können. Andere Verfahren sind jedoch ebenfalls von der Erfindung umfasst.

### Aufbringen von reduziertem Graphenoxid auf die Arbeitselektrode(n)

Die elektrophoretische Abscheidung von rGO bietet die Möglichkeit, günstig, einfach und fokussiert rGO auf der oder den Arbeitselektroden abzuscheiden.

Bevor das rGO elektrophoretisch abgeschieden wird, wird es erfindungsgemäß mit vorzugsweise biokompatiblen Kationen, insbesondere 2-wertigen Kationen wie Mg²⁺versetzt. Diese werden vom Graphen adsorbiert und/oder können ebenso interkalieren. Dadurch wird das Oberflächenpotential der einzelnen Graphen-Flakes, auch als Zeta-Potential bezeichnet, erhöht. Dieses Oberflächenpotential macht eine elektrophoretische Abscheidung erst möglich. Nach der Abscheidung bildet die konzentrierte Ansammlung der eingelagerten Kationen mit entsprechenden Gegenionen eine schichtförmige Substanz, z.B. Mg(OH)₂ im Falle der Verwendung von Mg²⁺-Ionen, die mit OH⁻-Ionen reagieren. Diese kommen aus dem (wässrigen oder wasserhaltigen) Lösungsmittel oder aus separat zugesetztem Wasser. Diese Schicht ist sehr fest und haftend und dient daher gleichzeitig als Haftschicht /Haftvermittler zwischen Graphen und der Elektrodenoberfläche. Weiterhin können auch andere Additive ergänzt werden, beispielsweise Cellulose, ein (anorganisches, organisches oder anorganischorganisches) Polymer oder Kautschuk oder eine Mischung mehrerer solcher Additive, um die Haftung zusätzlich zu erhöhen.

Das einfache Aufbringen von Graphen auf die Zielstruktur mittels elektrophoretischer Abscheidung bietet eine sehr günstige und einfache Herstellungsweise.

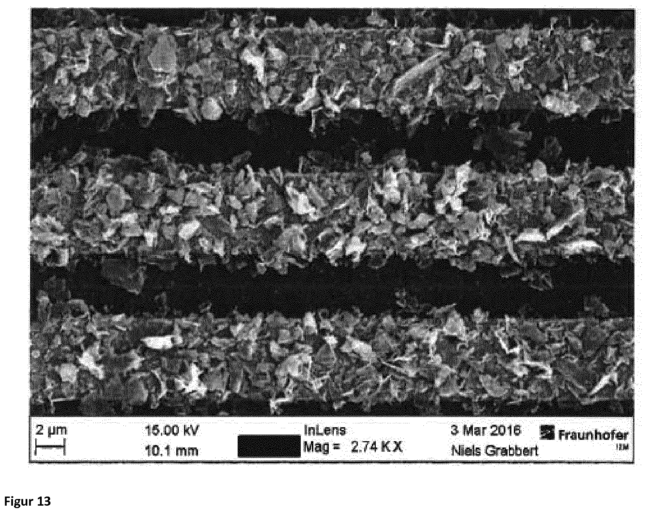

Figur 13 zeigt eine REM-Aufnahme einer IDE mit abgeschiedenem Graphen. Man erkennt eine dichte Belegung mit einer Vielzahl von Flakes. Hierdurch wird die Oberfläche der Elektroden stark erhöht.

Nach der elektrophoretischen Abscheidung haften Graphen-Flakes statistisch verteilt an der Elektroden-Oberfläche. Durch diese Orientierung wird die aktive Sensoroberfläche aus rGO extrem vergrößert, wie sich auch aus Fig. 13 ersehen lässt. An diese werden zur weiteren Erhöhung der Sensitivität während der weiteren Verfahrensschritte Fabs anstelle der größeren Antikörper kovalent gebunden. Die oben erläuterten einzigartigen elektrischen Eigenschaften des Graphens führen in Kombination mit dieser doppelten Erhöhung der Sensitivität zu einer einzigartigen Empfindlichkeit der möglichen Messmethoden.

Das rGO kann vor dem Aufbringen auf die Sensorbasis oder danach mit dem/den Abstandshalter(n) modifiziert werden. Bevorzugt ist die Modifikation vor dem Aufbringen.

Die in der vorliegenden Erfindung genutzten rGOs werden durch Umsetzen mit den Abstandshaltern funktionalisiert. Hierbei werden bevorzugt NH₂-PEG-NH₂ und/oder TEPA eingesetzt, wie oben beschrieben.

In einer Ausführungsform der Erfindung kann die Oberfläche des Graphens durch elektrophoretische Abscheidung von gefalteten Graphen-Flocken auf folgende Weise durch den Schritt (a) und/oder den Schritt (b) deutlich erhöht werden:
(a) Graphen-Flocken (rGO) werden in einem vorzugsweise organischen Lösemittel dispergiert. Die Dispergierung erfolgt vorzugsweise durch Ultraschallbehandlung, beispielsweise mittels Sonotrode. Auf diese Weise werden aggregierte rGO-Flocken aufgelöst bzw. vereinzelt und dann homogen dispergiert. Die rGO-Flocken werden durch die intensiven akustischen Wellen verformt bzw. gefaltet, wodurch eine dreidimensionale Struktur der zweidimensionalen rGO-Flocken erreicht wird. Die Graphenflocken haben eine Größe (maximale Länge) von 0,2 bis 3,0 µm.
(b) Die gefalteten rGO-Flocken werden nachfolgend auf die Arbeitselektrode abgeschieden. Dies erfolgt vorzugsweise durch elektrophoretischen Abscheidung (EPD). Die gefalteten rGO flocken zeichnen sich durch eine signifikante Vergrößerung Ihrer Oberfläche aus. Die Oberflächenvergrößerung erhöht die Sensitivität des Biosensors maßgeblich.

Durch das Herstellungsverfahren, das den Schritt (a) und/oder den Schritt (b) einsetzt, ist ein Biosensor erhältlich, der ohne diese Schritte eine wesentlich geringere Anzahl von Fängermolekülen pro Flächeneinheit der Arbeitselektrode aufweist und/oder eine wesentlich geringere Anzahl von Biomarkern pro Flächeneinheit der Arbeitselektrode binden kann. Ein bevorzugter Faktor der Erhöhung der Anzahl der gebundenen Fängermoleküle und/oder der Anzahl der Biomarker, die gebunden werden können, ist mindestens 1,2, stärker bevorzugt mindestens 1,5, noch stärker bevorzugt mindestens 2,0, noch stärker bevorzugt mindestens 3,0 und am stärksten bevorzugt mindestens 5,0. Dieser Faktor kann bestimmt werden, indem das gleiche Verfahren einmal mit und einmal ohne Schritt (a) und/oder Schritt (b) durchgeführt wird und die Anzahl der Fängermoleküle und/oder Biomarker pro Flächeneinheit bestimmt wird. Die Bestimmung dieser Anzahl bzw. des Unterschiedsfaktors kann über die detektierbare Funktion oder Eigenschaft der Fängermoleküle und/oder Biomarker erfolgen. Das heißt, es wird beispielsweise eine detektierbare Eigenschaft, z. B. Fluoreszenz, des Biomarkers einmal mit und einmal ohne Durchführung von Schritt (a) und/oder Schritt (b) gemessen und das Verhältnis der Werte als der vorstehend genannte Faktor angenommen.

Am Produkt, also am hergestellten Biosensor, lässt sich dieses Herstellungsverfahren durch REM-Untersuchung der Graphenschicht nachweisen. Bei der erfindungsgemäßen dreidimensionalen Strukturierung von Graphen ist zumindest ein Teil der flächigen Graphenflocken nicht in der Ebene der Oberfläche angeordnet, auf der die Graphenflocken abgeschieden wurden. Vielmehr nehmen die Flächen dieser Graphenflocken jeden möglichen Winkel zu der Ebene der genannten Oberfläche ein. Dieser Teil der Graphenflocken ist mindestens 50 % der gesamten Graphenflocken.

In einer besonders bevorzugten Ausführungsform ist die Anzahl der Fängermoleküle und/oder der Biomarker pro Flächeneinheit der Arbeitselektrode durch die dreidimensionale Strukturierung der Graphenflocken höher als die bei einer zweidimensionalen Strukturierung theoretisch mögliche Anzahl, in einer noch stärker bevorzugten Ausführungsform um den Faktor von mindestens 1,1, 1,2, 1,5, 2,0 oder 3,0 höher als die bei einer zweidimensionalen Strukturierung theoretisch mögliche Anzahl. Auch hier kann die Bestimmung dieser Anzahl bzw. des Unterschiedsfaktors über die detektierbare Funktion oder Eigenschaft der Fängermoleküle und/oder Biomarker erfolgen.

Durch die Ultraschallbehandlung werden die Graphenflocken verformt, d.h. die Flockenstruktur ist keine ebene Fläche mehr. Es ist bevorzugt, dass der Anteil dieser verformten Flocken an der Gesamtzahl der Flocken auf einer Arbeitselektrode, bestimmt durch REM-Analyse, mindestens 10 %, 20 %, 30 % , 50 % oder 70 % ist.

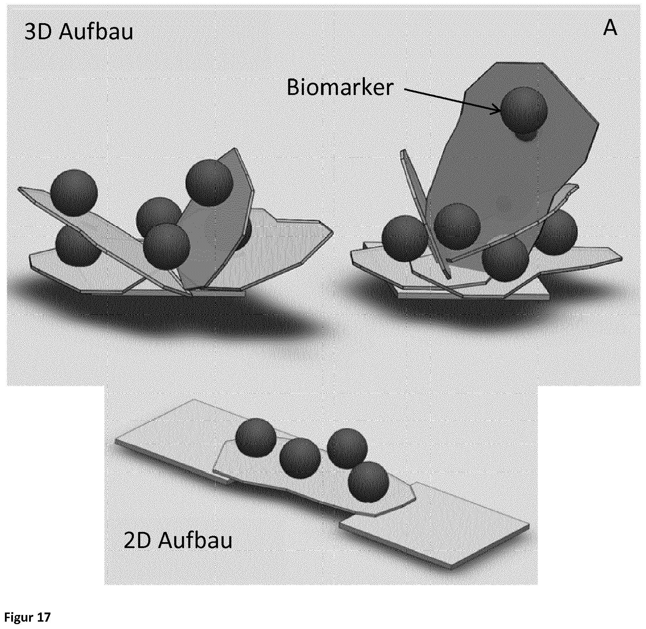

Figur 17 zeigt schematisch die Erhöhung der Oberfläche durch Erzeugung von dreidimensionalen Strukturen aus zweidimensionalen rGO-Flocken.

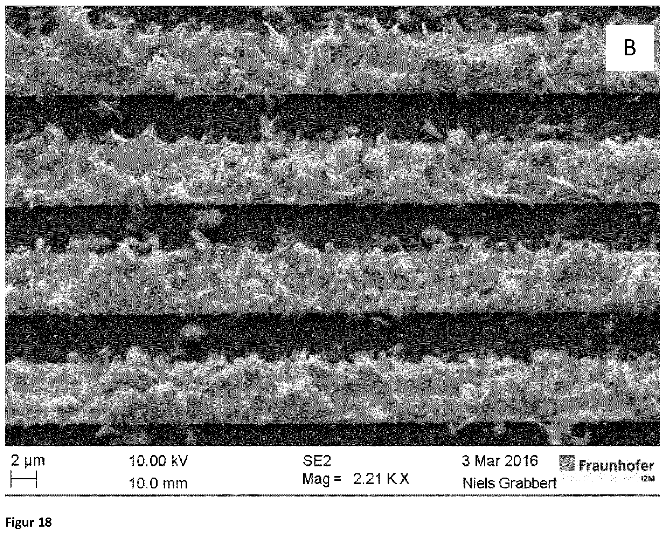

Figur 18 zeigt ein REM-Bild von dreidimensional angeordneten Graphenflocken.

### Anbindung der Fab-Fragmente oder anderer Fängermoleküle

Das freie Amino-Ende der Funktionalisierung des PEG-NH₂ oder TEPA eignet sich zum Anbinden an das Fab. Möglich ist es jedoch auch, einen COOH-Rest als Funktionalisierung am rGO zu nutzen. Denn Antikörper können auch über kovalente Bindungen an rGO-COOH-Gruppen angebunden werden. Da Fab-Fragmente im Wesentlichen aus Aminosäuren aufgebaut sind, besitzen sie sowohl freie -COOH als auch freie -NH₂-Gruppen. Deswegen sind sowohl Carboxy- als auch Aminreste am rGO als Bindungsterminal zum Fab möglich, um eine Amidbindung zu bilden. Bevorzugt sind jedoch freie -NH₂-Gruppen. Denn bei der Nutzung von Ig (Immunglobulinen) und daraus produzierten Fab-Fragmenten befinden sich N-Termini (NH₂-Reste) an der Antigenbindungsstelle der leichten und schweren Kette. Die gegenüberliegende Seite der Antigenbindungsstelle endet mit C-Termini (COOH-Rest). Um das Fab mit der richtigen Orientierung an das rGO zu binden, sollte das rGO daher mit -NH₂ funktionalisiert sein. So kann die komplementäre COOH-Gruppe des Fab-Fragments an das rGO binden und die richtige Orientierung sichergestellt werden.

Die Kombination der Verwendung von rGO und der beschriebenen Funktionalisierung hat den Vorteil, dass das mit Abstandshalter funktionalisierte rGO, z.B. NH₂-PEG-rGO oder TEPA-rGO, als bioelektrisches Interface mit hoher Biokompatibilität von NH₂-PEG-rGO und TEPA-rGO eingesetzt werden.

Wie bereits oben ausgeführt, kann für die Funktionalisierung beispielsweise Tetraethylenpentamin (TEPA) der Formel (1)

H₂N-CH₂CH₂-NH-CH₂CH₂-NH-CH₂CH₂-NH-CH₂CH₂-NH₂ (Formel (1))

eingesetzt werden. Käuflich erwerbbares TEPA ist häufig ein Gemisch aus mehreren Verbindungen (Oligomeren), die aus einer unterschiedlichen Monomerzahl entstanden sind und daher eine unterschiedliche Kettenlänge aufweisen. Das in der vorliegenden Verbindung verwendete TEPA kann daher z.B. eine oder mehrere der Verbindungen enthalten, die aus der Gruppe ausgewählt sind, die aus N-(2-Aminoethyl)-N'-{2-{2-aminoethyl)amino}ethyl}-1,2-ethan-diamin; 4-(2-Aminoethyl)-N-(2-aminoethyl)-N-{2-{(2-aminoethyl)amino}ethyl}-1,2-ethandiamin; 1-(2-Aminoethyl)-4-[(2-aminoethyl)amino]ethyl]piperazin) und 1-[2-[[2-[(2-Aminoethyl)amino]ethyl]amino]ethyl]piperazin) besteht.

Diese Verbindungen unterscheiden sich in ihrer Struktur und damit ihrer Reaktivität mit den Reaktionspartnern, nämlich reduziertem Graphen einerseits und Antikörperfragment Fab oder einem anderen Fängermolekül andererseits.

Somit umfasst der hier verwendete Ausdruck "Tetraethylenpentamin" oder "TEPA" nicht nur die Verbindung der Formel (1), sondern auch jede der anderen vorstehend genannten Verbindung allein oder in einem Gemisch von zwei oder mehreren dieser Verbindungen.

Das in der vorliegenden Erfindung verwendete Polyethylenglycol-NH₂ (PEG-NH₂) leitet sich von Polyethylenglycol (PEG) ab. PEG ist ein Polymer aus Ethylenglycol-Monomereinheiten und kann unterschiedlichste Kettenlängen aufweisen. Typische Kettenlängen von käuflich erwerbbarem PEG umfassen 200, 400, 600 oder 1500 Monomereinheiten. In der vorliegenden Erfindung kann das PEG-NH₂ aus 5 bis ca. 3500, vorzugsweise aus 10 bis 600, stärker bevorzugt aus 20 bis 100 der Monomereinheiten bestehen. Es kann PEG-NH₂einer einzigen Kettenlänge oder PEG-NH₂ verschiedener Kettenlängen eingesetzt werden. Das heißt, die vorliegende Erfindung umfasst auch eine Ausführungsform, in der zur Herstellung des Biosensors ein Gemisch aus PEG-NH₂ verschiedener Kettenlänger eingesetzt wird. Zusätzlich kann dieses Gemisch auch ein oder mehrere der vorstehend genannten verschiedenen TEPAs enthalten.

Auf diese Weise können Abstandshalter verschiedener Länge und auch Reaktivität auf dem reduzierten Graphen eines Biosensors erhalten werden.

Die Verwendung von Abstandshaltern verschiedener Länge hat den Vorteil, dass die sensorische Oberfläche erhöht werden kann. Konkret bedeutet dies, dass auf dem reduzierten Graphen die Antikörperfragmente Fab bzw. andere Fängermoleküle quasi in mehreren Schichten und trotzdem gut zugänglich vorhanden sein können.

In einer Ausführungsform der vorliegenden Erfindung können die NH₂-rGO-Termini mittels Azidoacetylchlorid azidiert werden. Durch diese Funktionalisierung kann Klick-Chemie als Bindungsmechanismus zwischen rGO und Fängermolekül genutzt werden. Über die Klick-Chemie können die verschiedensten Moleküle an die rGO Oberfläche gebunden werden. Dies kann ohne die Zugabe zusätzlicher Reaktionschemie erfolgen. Beispielsweise können auf diese Weise L-Aptamere erfolgreich als Antikörperäquivalent an rGO gebunden werden.

Aptamere bestehen aus synthetisch hergestellten DNA/RNA Fragmenten, welche durch Ihre spezifische Raumordnung hochselektiv an definierte Antigene binden. Diese Bindung ist mit der Bindung von Antikörpern an Antigene vergleichbar. Die Bindungsaffinitäten sind ebenfalls vergleichbar mit Antikörpern. Im Vergleich zu Proteinen (Antikörper/FABs) sind Moleküle aus DNA/RNA (Aptamere) wesentlich stabiler gegenüber Temperatur und Chemikalien. Dadurch wird verglichen mit anfälligen Antikörpern eine einfachere Integration innerhalb diverser Herstellungsverfahren möglich.

L-Aptamere sind eine besondere Gattung der Aptamere. Sie zeichnen sich durch eine linksdrehende Chiralität aus. Hierdurch wird eine enorm erhöhte Biostabilität erreicht, da DNA/RNA-Polymerasen nicht mehr in der Lage sind, die DNA/RNA-Anteile der Aptamere abzubauen.

Eine Verwendung von vollsynthetisch hergestellten Fängermolekülen erlaubt außerdem die Vermeidung von Tiermodellen und ermöglich gleichzeitig eine erhöhte Qualität der Aptamere im Vergleich zu Antikörpern.

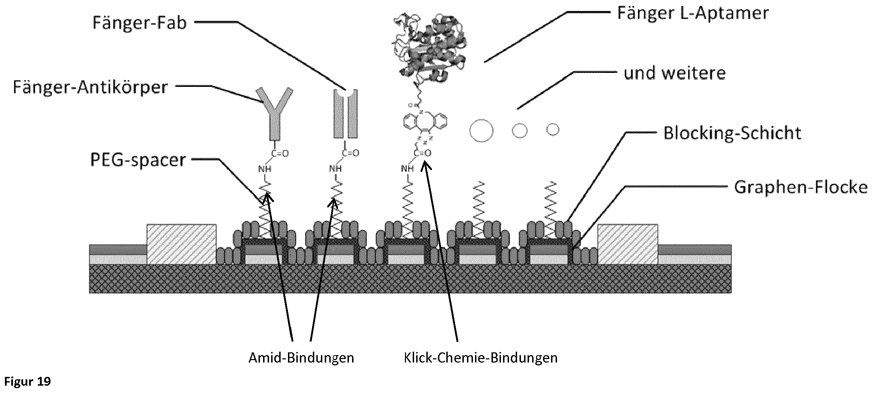

Figur 19 ist eine schematische Darstellung der Funktionalisierung mittels Klick-Chemie und Amidbindung.

Die Kombination von Klick-Chemie und Amidbindung zur Funktionalisierung kann vorteilhaft sein, beispielsweise zur Erzeugung verschiedener Selektivitäten auf einem Sensor.

In besonders vorteilhafter Weise kann die vorstehend beschriebene Herstellung einer dreidimensionalen Strukturanordnung mit der Funktionalisierung mittel Klick-Chemie und/oder Amidbindung kombiniert werden.

### Antikörperfragmente Fab

Einen besonderen Vorteil bietet die Erfindung durch die Verwendung von Antikörperfragmenten Fab anstelle von ganzen Antikörpern, da Fab-Fragmente kleiner sind, linear bzw. langgestreckt sind und nicht die Y-Form von Antikörpern aufweisen.

Durch die Kombination der Verwendung von Fab-Fragmenten und verschieden langen Abstandshaltern kann somit nicht nur die Anzahl der Fab-Fragmente pro Flächeneinheit, sondern auch die Anzahl der Fab-Fragmente pro Höheneinheit erhöht werden. Dadurch kann die Anzahl der Fab-Fragmente pro Volumeneinheit drastisch erhöht werden.

Der erfindungsgemäße Biosensor nutzt nur den sensitiven Teil eines Antikörpers. Damit kann im Vergleich zur Nutzung des gesamten Antikörpers ein besseres Gewichts- und Volumenverhältnis zwischen Fab-Fragment und Biomarker realisiert werden. Berechnungen ergeben, dass der effektive Radius eines Antikörpers 5 nm beträgt, während derjenige des Fab-Fragments 1,25 nm beträgt. Dies ergibt bei Annahme eines Kreises eine Fläche des Antikörpers von 78 nm² und des Fab-Fragments von 4,91 nm². Da ein Antikörper auf dieser Fläche zwei Bindungsstellen aufweist, ist das Verhältnis von Bindungsstellen pro Flächeneinheit bei einem Fab-Fragment achtmal höher als bei einem Antikörper.

Als Beispiel kann die Bindung des Antigens Procalcitonin (PCT) an IgG-Antikörper einerseits und Fab-Fragment andererseits betrachtet werden. Die Masse eines IgG-Antikörpers beträgt 150 kDa, die Masse eines Fab-Fragments beträgt 46 kDa. Die Masse von Procalcitonin (PCT) beträgt 13 kDa. Bei Bindung von PCT an einen Antikörper ergibt sich ein Massenverhältnis (2xPCT)/Antikörper von 17,3 %, während sich bei Bindung von PCT an ein Fab-Fragment ein Massenverhältnis von PCT/Fab von 28,2 % ergibt. Somit ermöglicht die Verwendung eines Fab-Fragments die Bindung einer wesentlich höheren Anzahl von Antigenen pro eingesetzter Masse. Das bessere Gewichts- und Volumenverhältnis führt zu höherer Sensitivität des Biosensors. Durch die kleine Fläche des Fab-Fragments im Vergleich zu der Fläche des gesamten Antikörpers kann mehr sensitives Material pro Flächeneinheit aufgebracht werden.

### Herstellung des Biosensors auf einem Chip

Im Folgenden wird anhand einer bevorzugten Ausführungsform die Herstellung des erfindungsgemäßen Biosensors auf Chipebene beschrieben. Es sollte aber klar sein, dass alle Schritte auch in anderen Kombinationen möglich sind.

In einem ersten Schritt wird mit üblichen Techniken wie Dünn- oder Dickschichttechnologie ein Sensorkörper erzeugt, der aus einem nichtleitenden Substrat besteht, auf dem mindestens eine Arbeitselektrode abgeschieden wird, die üblicherweise aus einem geeigneten, biokompatiblen Metall besteht. Zwischen dem Substrat und der/den Arbeitselektrode(n) kann/können eine oder mehrere geeignete Zwischenschichten wie z.B. eine Haftschicht angeordnet sein. Die Ebene der Arbeitselektrode(n) kann auch zur Ausbildung von Ankontaktierungen genutzt werden; alternativ können die Ankontaktierungen im Substrat vergraben sein.

Neben der/den Arbeitselektroden kann eine Gegenelektrode ausgebildet sein, die ggf. ebenfalls auf derselben Ebene wie die Arbeitselektrode(n) angeordnet und in geeigneter Weise ankontaktiert wird.

Soll eine Substrat-Gate-Elektrode vorhanden sein, so wird diese nach bekannten Methoden im isolierenden Substrat ausgebildet und ankontaktiert.

Soll eine Referenzelektrode vorhanden sein, kann diese an geeigneter Stelle, beispielsweise auf dem Substrat oder einer Haftschicht, aufgedruckt werden, beispielsweise als Ag/AgCl-Paste.

Auf dem Substrat befindliche Kontaktstrukturen, die zu den Elektroden führen, können mit einem isolierenden Material, beispielsweise einem Kunststoff, abgedeckt werden. Dieser kann so aufgebracht werden, dass die Arbeitselektroden in Aussparungen dieses Materials zu liegen kommen, derart, dass eine zu detektierende Flüssigkeit als Tropfen oder dgl. darin liegen bleibt und nicht weglaufen kann.

Die Arbeitselektroden, vorzugsweise Interdigitalelektroden, werden mit reduziertem Graphenoxid (rGO) beschichtet, welches mit einem geeignetem Abstandshalter Spacer wie oben beschrieben, z.B. mit aminierten-Polyethylenglycol (NH₂-PEG-rGO) und/oder mit Tetraethylenpentamin (TEPA-rGO), funktionalisiert ist. Vor der Beschichtung wird das Graphen, vor oder nach der Funktionalisierung mit dem Spacer, mit vorzugsweise 2-wertigen, biokompatiblen Metallkationen, insbesondere mit Mg²⁺-Ionen, aufgeladen, die dabei interkalieren und beim Aufbringen auf die Elektroden in Form des Hydroxids eine Haftschicht bilden.

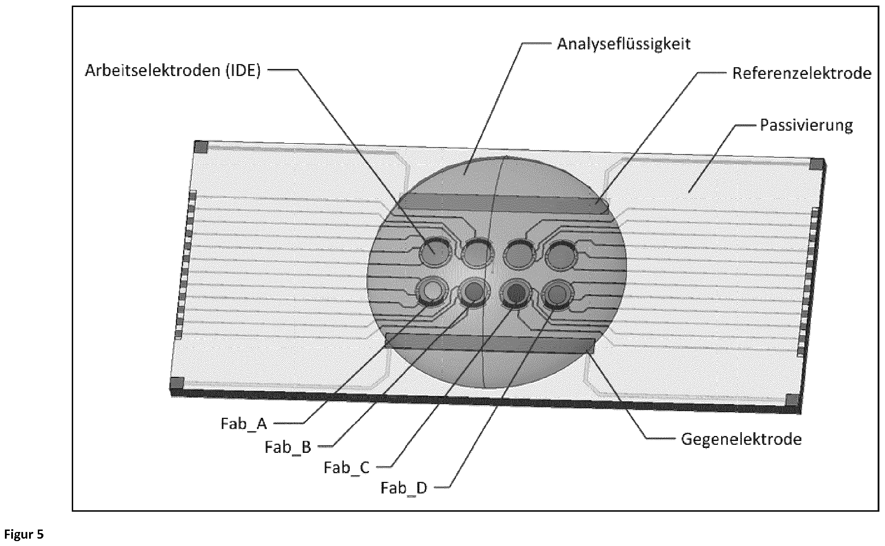

Anschließend werden definierte Antikörper-Fab-Fragmente bzw. andere Fängermoleküle kovalent an die Aminogruppen der Abstandshalter (Spacer) am reduzierten Graphenoxid gebunden. Das Fab-Fragment legt hierbei die Selektivität zu einem speziellen Analyten ("Biomarker") fest und kann dementsprechend auf den Wunschanalyten bzw. -biomarker angepasst werden. Es ist eine Anordnung von mehreren Interdigitalelektroden auf einem Chip möglich. Diese können gleiche oder unterschiedliche Fab-Fragment-Funktionalisierung aufweisen (Figur 5).

Nach der Funktionalisierung kann eine Sperrschicht (Blocking-Layer) zur Eliminierung von freien aktiven Oberflächen abgeschieden werden. Gleichzeitig reduziert diese Schicht die parasitäre Wechselwirkung durch Sättigung der freien Bindungen auf den Oberflächen und die Unterdrückung von unspezifischer Biomarker-Bindung.

Es kann eine zusätzliche Stabilisierungsschicht aufgebracht werden, um die Lebenszeit und Zuverlässigkeit des Biosensors zu verbessern. Diese kann beispielsweise aus Zucker bestehen, der sich bei Nutzung des Biosensors wieder löst.

Die kovalente Bindung zwischen Graphen und Antikörper-Fab-Fragment ermöglicht eine höhere Mobilität des Fab-Fragments im Vergleich zu adsorbierten Fab-Fragmenten. Dadurch ist eine bessere Aufbringung von Sperrschichten (Blocking-Layer) ohne Beeinträchtigung der Fab-Fragmente gewährleistet. Diese Blockierung führt zu einer Reduzierung von parasitären Wechselwirkungen. Außerdem widersteht die starke kovalente Bindung zwischen Graphen und Fab-Fragmenten Reinigungsprozessen, wodurch eine Mehrfachnutzung des Sensors ermöglicht wird.

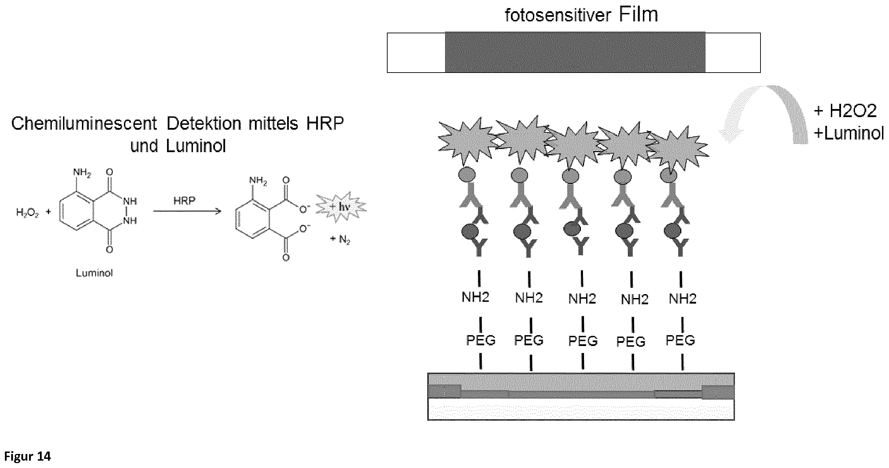

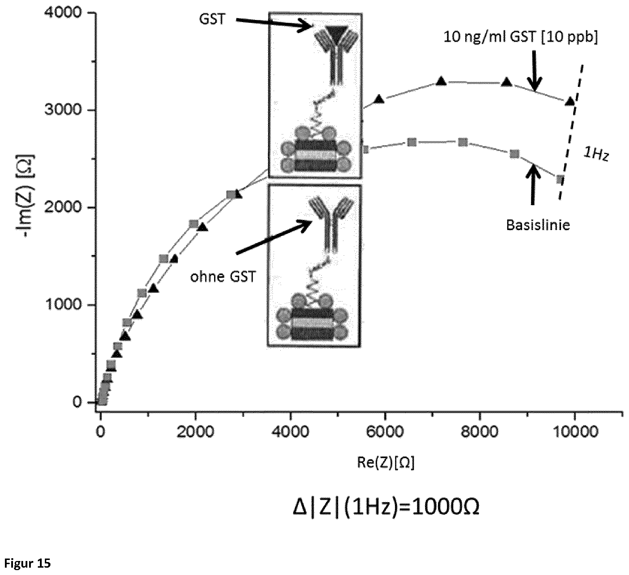

Die kovalente Bindung an das Graphen wurde vorliegend der Einfachheit halber nicht mit einem Fab-Fragment, sondern mit einem Antikörper (IgG-Anti-GST, polyklonal) nachgewiesen. Die Bindung zwischen Antikörper und Graphen konnte über Chemolumineszenz-Detektion nachgewiesen werden. Hierbei wurde GST (Glutathion-S-Transferase) als Biomarker genutzt, der mittels Sandwich-ELISA detektiert wurde (Figuren 14 und 15).

### Messung der Biomarkerbindung

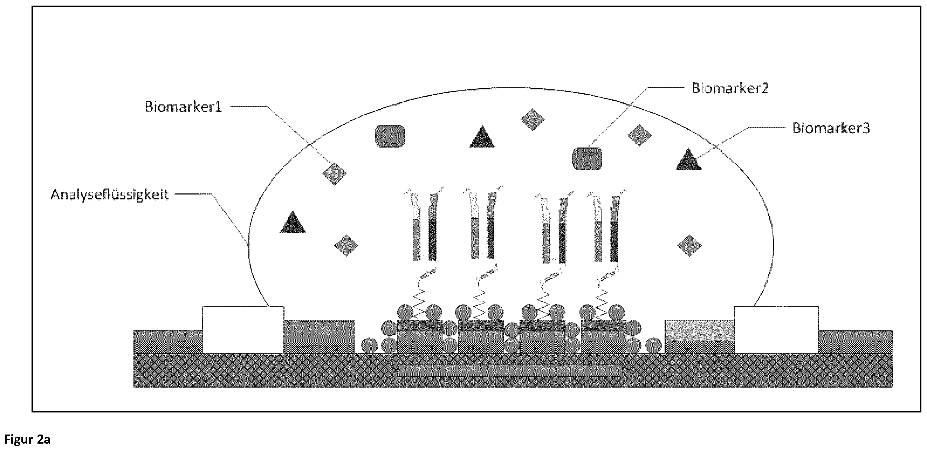

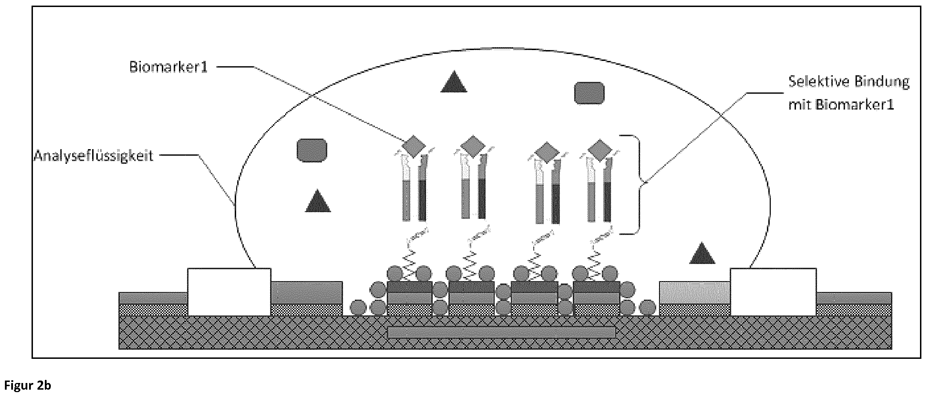

Der Biosensor registriert die verschiedenen Konzentrationen von Biomarkern durch Veränderung seiner elektrischen Eigenschaften. Eine Änderung der Biomarkerkonzentration (Figuren 2a und 2b) ruft hierbei mindestens eine messbare Änderung hervor, die aus der Gruppe ausgewählt ist, die aus einer messbaren Änderung des elektrischen Stroms, der elektrischen Spannung, der elektrischen Kapazität, der elektrischen Induktivität, des elektrischen Widerstands und der elektrischen Impedanz des Biosensors besteht.

### Beispiele

Die vorliegende Erfindung wird anhand des nachfolgenden Beispiels weiter veranschaulicht.

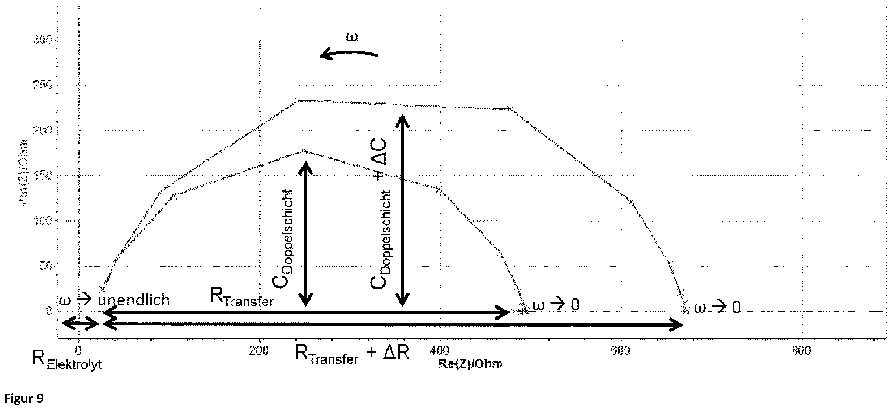

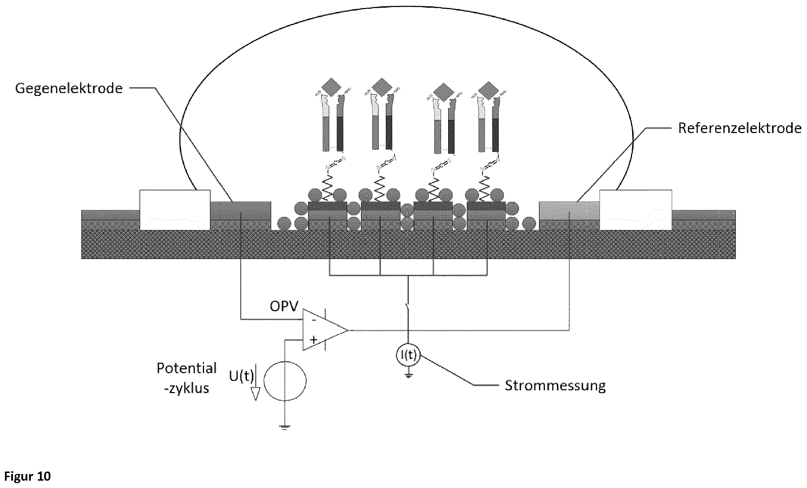

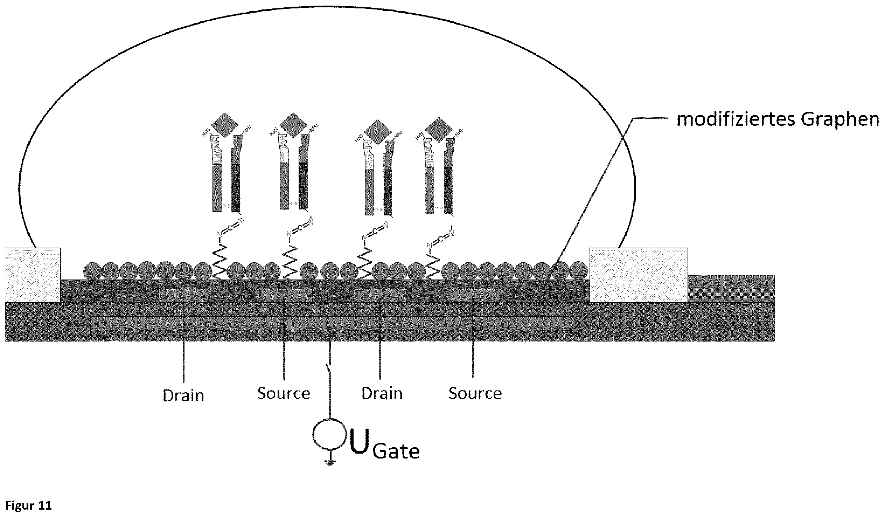

Ein Biosensor wird durch die folgenden Schritte (1) bis (5) hergestellt:
(1) Herstellung einer Arbeitselektroden-Struktur
   Eine oder mehrere IDE-Elektrodenstrukturen werden aus Gold oder einem anderen geeigneten Metall mittels Standard-Halbleitertechnologie auf einem nichtleitenden Substrat aufgebracht.
(2) Herstellung einer elektrophoretisch abscheidbaren rGO-PEG-NH₂-Suspension mittels Ultraschall. Zunächst wird rGO-PEG-NH₂ in einer Konzentration von einigen mg/ml zu dielektrischem Lösemittel (z.B. Ethanol, Isopropanol, Aceton, NMP (N-Polymethylpyrrolidon), Dimethylformamid (DMF)) gegeben. Die Mischung wird mehrere Male mittels Sonotrode beschallt. Danach wird die Oberfläche des rGO-PEG-NH₂ durch Zugabe von MgCl₂-6H₂0 mit Kationen aufgeladen (Mg²⁺ wird an rGO-PEG-NH₂ adsorbiert und/oder in die Struktur interkaliert). Bei Bedarf können PVDF, PE, PMMA oder andere Polymere oder anorganische und oder andersartige organische Stoffe als Haftvermittler dazugegeben werden. Die Lösung wird über einige Stunden mittels Sonotrode ("Ultraschall-Mixer") dispergiert. Die erhaltene Dispersion wird bei >2000 rpm zentrifugiert, und der Überstand wird von Sedimenten getrennt. Der Überstand wird mittels Spritzenfilter (<10 µm) filtriert.
(3) Elektrophoretische Abscheidung von rGO-PEG-NH₂ auf die Arbeitselektrode Eine IDE aus Gold wird als Arbeitselektrode genutzt (negatives Potential). Es wird eine inerte Gegenelektrode (positives Potential) verwendet. Beide Elektroden werden in die bereits vorbereitete rGO-PEG-NH₂-Lösung getaucht; das Schichtwachstum wird über die angelegte Spannung, Abscheidezeit, den Elektrodenabstand und die -fläche sowie über unterschiedliche rGO-PEG-NH₂ und MgCl₂-6H₂0-Konzentrationen der Lösung variiert; die Abscheidung erfolgt entweder im Gleichspannungsmodus oder im positiv gepulsten Spannungsmodus; am rGO angelagerte Mg²⁺-Ionen bilden nach gewisser Zeit mit im System vorhandene OH⁻-Ionen die Verbindung Mg(OH)₂. Eine Matrix aus Graphen-Mg(OH)₂ konnte mittels REM und EDX dokumentiert werden (Figur 13).
(4) Aushärten
   Hierzu wird der Chip nach der Abscheidung in einen Vakuumofen gelegt und mehrere Stunden ausgebacken. Die Lösemittelreste werden so aus der Oberfläche gezogen und es kommt zu einer Verdichtung der Schicht. Mg(OH)₂ bildet mit rGO-PEG-NH₂ und der Elektrodenoberfläche eine feste, haftende Schicht. Bei Bedarf kann diese Schicht durch hierin genannte Haftvermittler optimiert werden.
(5) Bindung von Fab-Fragmenten an rGO-PEG-NH₂
   Fab-Fragmente werden in 1:10 PBS (phosphatebufferedsaline) dispergiert. NHS (N-Hydroxysuccinimid) und EDC (1-Ethyl-3-[3-dimethylaminopropyl]carbodiimid) werden ebenfalls in 1:10 PBS gelöst. Anteile der NHS/EDC-Lösung werden zur Fab-Fragmente Lösung gegeben, welche einige Minuten bei Raumtemperatur miteinander reagieren. Die zur Reaktion gebrachte Lösung wird nun auf die rGO-PEG-NH2-Oberfläche des Chips pipettiert. Dort bilden die aktivierten Fab-Fragmente mit dem rGO-PEG-NH₂ stabile kovalente Verbindungen. Der Ansatz wird mittels 1:10 PBS von der Chipoberfläche gewaschen. Danach wird ein Blocking-Buffer (z.B. aus Milchpulver/1:10PBS/0,1% Tween20) auf die Sensoroberfläche gegeben und einige Zeit inkubiert. Überschüssiger und nicht gebundener Blocking-Buffer wird unter Verwendung von Wasch-Buffer (z.B mit 1:10 PBS oder 1:10 PBS/0,1% Tween20) entfernt.
(6) Elektrische Messmethoden
   Alle Messungen erfolgen in einer Test- oder Analytlösung, der ein Elektrolytsalz zugegeben wird.
   a. Elektrochemische Impedanzspektroskopie
      Hierbei wird in dem Messsystem ein definiertes Wechselsignal auf die Interdigitalelektroden gegeben und das Outputsignal gemessen, um die Impedanz (Z) des Messsystems zu charakterisieren. Die Impedanz wird oft im Frequenzbereich Z(ω) betrachtet.
      Zur einfacheren Charakterisierung des Systems wird Z(ω) in der Komplexenebene geplottet. Es können nun die einzelnen elektrischen Komponenten aus dem Plot extrahiert sowie Änderungen leicht erkannt werden. Die Figuren 8 und 9 zeigen ein vereinfachtes elektrisches Ersatzschaltbild eines IDE-Fingers ohne (Fig. 8) und mit (Fig. 9) Biomarkerbindung in Elektrolytlösung.
   b. Zyklische Voltammetrie (CV)
      Während der CV-Messung ist es günstig, ein Red-Ox-Additiv in die Messlösung zu integrieren (z.B. K₃Fe(CN)₆/ K₄Fe(CN)₆), wenn die Elektrodenoberfläche nicht selbst ein Red-Ox-System bildet.
      Während der Messung mit Hilfe zyklischer Voltammetrie wird das zugesetzte Red-Ox-System zyklisch oxidiert und reduziert. Dadurch wird das Potential der Arbeitselektrode im Verhältnis zu einer Referenzelektrode definiert verändert. Gleichzeitig wird der geflossene Strom gemessen. Red-Ox-Reaktionen, die durch diese Potentialzyklen ausgelöst werden, sind durch einen erhöhten Stromfluss später klar erkennbar. Da der Stromfluss von der Elektrodenoberfläche abhängt und sich diese Elektrodenoberfläche bei der Anlagerung von Antigenen an die Antikörper verändert (der Ladungstransfer von Ion zu Elektrode und umgekehrt wird dadurch verändert), kann die Anlagerung der Antigene auf diese Weise detektiert werden: Es ergibt sich ein anderes CV-Bild als in Abwesenheit der Antigene.
      Hierbei ergeben sich 2 Optionen für die Messstrategie.
      1. Es existiert nur eine mit Fab belegte Arbeitselektrode. Im Falle des Vorhandenseins von Interdigitalelektroden werden diese auf das gleiche Potential gelegt und bilden die Arbeitselektrode, welche in einem Elektrolyten gegen eine Referenzelektrode (z.B. Ag/AgCl₂) vermessen werden. Die Referenzelektrode kann mittels Ag/AgCl₂-Pastendruck auf Chiplevel integriert werden.
      2. Alternativ kann eine der Interdigitalelektroden als Pseudo-Referenz und die andere als Arbeitselektrode dienen.
   c. Liquid "Gate" CV
      Bei diesem Verfahren dient die Testflüssigkeit als Elektrolyt. Wiederum ist es günstig, ein Red-Ox-Additiv in die Messlösung zu integrieren (z.B. K₃Fe(CN)₆/ K₄Fe(CN)₆), wenn die Elektrodenoberfläche nicht selbst ein Red-Ox-System bildet. Über die Referenzelektrode wird der CV-typische Potentialzyklus (über den Elektrolyten) in das System eingeprägt, und die geflossenen Ströme werden über die Elektroden (mit Fabs belegte Elektrode(n) gegen die Gegenelektrode oder die Referenzelektrode) gemessen. Wie vorstehend beschrieben wird auch hier das eingeprägte Potential gegen den geflossenen Strom aufgetragen. Dabei ergibt sich eine ähnliche Red-Ox-Charakteristik wie im Verfahren b.
      Um das Potential in der Flüssigkeit auf dem gewünschten Niveau zu halten (trotz ausgelösten Red-Ox-Reaktionen), kann die Referenzelektrode mit einem Operationsverstärker (OPV) und einer Gegenelektrode ausgestattet werden. Der OPV vergleicht das Potential an Gegenelektrode und Referenzelektrode mit dem Wunschpotential (Potentialzyklus) und regelt bei Abweichungen gegen (Figur 10). Hierbei wird ein potentionstatischer Aufbau erreicht wie aus dem Stand der Technik bekannt.
   d. Arbeitspunkteinstellung bzw. Sensitivitätsverbesserung mittels Gate-Anordnung
      Es besteht die Möglichkeit, die halbleitenden Eigenschaften des Graphens so auszunutzen, dass dadurch der Systemarbeitspunkt eingestellt werden kann und daraus eine höhere Sensitivität resultiert. Hierzu kann eine Gate-Elektrode in das Substrat eingebettet werden, welche mit einem bestimmten Potential angesteuert werden kann. Wie aus Fig. 11 ersichtlich, kann über U_{Gate} der Arbeitspunkt des Graphens eingestellt werden. Zwischen den Interdigitalelektroden (hier mit "Drain" und "Source" bezeichnet) wird der Strom oder die Spannung gemessen.

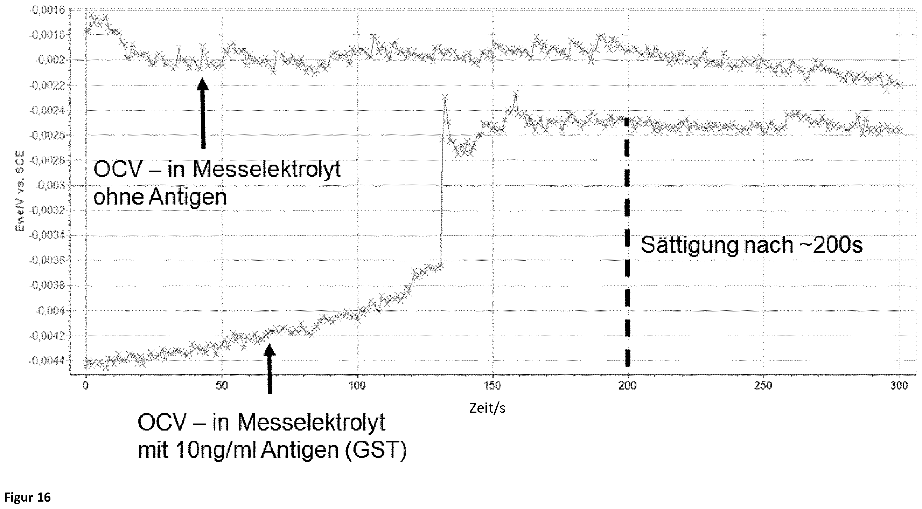

Außerdem kann eine OCV Messung, d.h. eine Messung unter Leerlaufspannung (open-circuit voltage) durchgeführt werden. Hierbei wird ein erster Elektrodenpfad (eine der mit Fabs belegten Interdigitalelektroden) als Null-Potential definiert und die Potentialdifferenz zum anderen Elektrodenpfad ermittelt. Dadurch kann eingeschätzt werden, ab wann sich das System in einem stabilen Zustand befindet. Falls das OCV stabil bleibt, findet keine Veränderung an der Oberfläche der Elektroden bzw. keine chemischen (oder nur signifikant geringe) Wechselwirkung zwischen Elektrolyten und Elektrode statt. Dadurch kann die Reaktionskinetik des detektierten Biomarkers (Analyten, Antigens) mit der Oberfläche sichtbar gemacht werden, da es hier zu einem Massentransport und Veränderung der Oberfläche kommt. Wenn die Oberfläche gesättigt ist, stabilisiert sich das OCV und der Biosensor kann vermessen werden. Damit wird auch gleichzeitig die Schnelligkeit des Sensors ermittelbar. Figur 16 zeigt das OCV eines Prototypen.

## Patentansprüche

1. Biosensor, der folgende Komponenten umfasst:
(a) eine Sensorbasis mit einem isolierenden Substrat und mindestens einer darauf befindlichen, elektrisch leitenden Arbeitselektrode,
(b) auf mindestens eine der Arbeitselektroden aufgebrachtes reduziertes Graphenoxid (rGO) in der Form von flächigen Flocken einer Größe, das heißt maximalen Länge, von 0,2 bis 3,0 µm, wobei die Flächen von mindestens 50 % der Graphenoxidflocken von der Ebene der Arbeitselektrodenfläche, auf der sie aufgetragen sind, abweichen,
(c) an das reduzierte Graphenoxid kovalent gebundene Abstandshalter ("Spacer") unterschiedlicher Länge,
(d) jeweils ein an die Abstandshalter kovalent gebundenes Fängermolekül.

2. Biosensor nach Anspruch 1, wobei das Fängermolekül ein Antikörperfragment Fab oder ein L-Aptamer ist.

3. Biosensor nach Anspruch 1 oder 2, bei dem die Komponenten (b) bis (d) auf eine Arbeitselektrode, wobei der Biosensor weiterhin eine Referenzelektrode aufweist, oder auf mit Wechselstrom beaufschlagbare Interdigitalelektroden aufgebracht sind.

4. Biosensor nach einem der vorstehenden Ansprüche, wobei als Abstandshalter endseitig aminierte Polyalkylenglycol-Moleküle unterschiedlicher Länge und/oder Polyalkylenpolyamin-Moleküle unterschiedlicher Länge dienen.

5. Biosensor nach einem der vorstehenden Ansprüche, worin die kovalente Bindung zwischen dem Abstandshalter und dem Fängermolekül durch Umsetzen einer Carboxylgruppe des Antikörperfragments Fab als Fängermolekül mit einer Aminogruppe des Abstandshalters oder durch Umsetzen einer mittels Azidoacetylchlorid azidierten Aminogruppe mit einem DNA- oder RNA-Molekül als Fängermolekül erhältlich ist.

6. Verfahren zum Herstellen eines Biosensors, welches die folgenden Schritte umfasst:
(i) das Bereitstellen einer Sensorbasis mit einem isolierenden Substrat und mindestens einer darauf befindlichen, elektrisch leitenden Arbeitselektrode,
(ii) das Bereitstellen von reduziertem Graphenoxid, das mit difunktionellen Aminoverbindungen unterschiedlicher Länge funktionalisiert wurde, in der Form von flächigen Flocken einer Größe, das heißt maximalen Länge, von 0,2 bis 3,0 µm,
(iii) das Auftragen des in Schritt (ii) bereitgestellten funktionalisierten reduzierten Graphenoxids auf die mindestens eine Arbeitselektrode mittels elektrophoretischer Abscheidung sowie
(iv) das Umsetzen eines Fängermoleküls mit dem in Schritt (iii) erhaltenen Produkt
wobei das reduzierte Graphenoxid zwischen den Schritten (ii) und (iii) in einem Lösungsmittel unter Ultraschallbehandlung dispergiert wird, und
wobei die Flächen von mindestens 50 % der Graphenoxidflocken von der Ebene der Arbeitselektrodenfläche, auf der sie aufgetragen sind, abweichen.

7. Verfahren nach Anspruch 6, wobei ein Biosensor nach einem der Ansprüche 1 bis 5 erhalten wird.

8. Verfahren zum Herstellen eines Biosensors, welches die folgenden Schritte umfasst:
(i) das Bereitstellen einer Sensorbasis mit einem isolierenden Substrat und mindestens einer darauf befindlichen, elektrisch leitenden Arbeitselektrode,
(ii) das Bereitstellen von reduziertem Graphenoxid in der Form von flächigen Flocken einer Größe, das heißt maximalen Länge, von 0,2 bis 3,0 µm,
(iii) das Funktionalisieren des reduzierten Graphenoxids mit difunktionellen Aminoverbindungen,
(iv) das Auftragen des bereitgestellten funktionalisierten reduzierten Graphenoxids auf die mindestens eine Arbeitselektrode mittels elektrophoretischer Abscheidung und
(v) das Umsetzen eines Fängermoleküls mit dem in Schritt (iv) erhaltenen Produkt,
wobei das reduzierte Graphenoxid zwischen den Schritten (ii) und (iii) oder zwischen den Schritten (iii) und (iv) in einem Lösungsmittel unter Ultraschallbehandlung dispergiert wird, und
wobei die Flächen von mindestens 50 % der Graphenoxidflocken von der Ebene der Arbeitselektrodenfläche, auf der sie aufgetragen sind, abweichen.

9. Verfahren nach Anspruch 8, wobei ein Biosensor nach einem der Ansprüche 1 bis 5 erhalten wird.

10. Biochip, der einen Biosensor nach einem der Ansprüche 1 bis 5 trägt.

11. Verfahren zum Nachweisen eines Analyten, welches das Kontaktieren eines in einem flüssigen Medium befindlichen Analyten mit einem Biosensor nach einem der Ansprüche 1 bis 5 oder mit einem Biochip nach Anspruch 10 und das nachfolgende Messen der durch die Wechselwirkung des Analyten mit dem Biosensor bzw. Biochip bewirkten Änderung einer elektrischen Eigenschaft des Biosensors umfasst.

## Claims

1. A biosensor comprising the following components:
(a) a sensor base comprising an insulating substrate and at least one electrically conductive working electrode thereon,
(b) reduced graphene oxide (rGO) deposited on at least one of the working electrodes in the form of sheet-like flakes having a size, i.e. maximum length, of 0.2 to 3.0 µm, wherein the faces of at least 50% of the graphene oxide flakes deviate from the plane of the working electrode surface on which they are deposited,
(c) spacers of different lengths covalently bonded to the reduced graphene oxide,
(d) one capture molecule covalently bonded to each of the spacers.

2. The biosensor of claim 1, wherein the capture molecule is an antibody fragment Fab or an L-aptamer.

3. The biosensor according to claim 1 or 2, wherein components (b) to (d) are applied to a working electrode, the biosensor further comprising a reference electrode, or to interdigital electrodes on which alternating current is applicable.

4. The biosensor according to any one of the preceding claims, wherein terminally aminated polyalkylene glycol molecules of different lengths and/or polyalkylene polyamine molecules of different lengths serve as spacers.

5. The biosensor according to any one of the preceding claims, wherein the covalent bond between the spacer and the capture molecule is obtainable by reacting a carboxyl group of the antibody fragment Fab as the capture molecule with an amino group of the spacer or by reacting an amino group azidated by azidoacetyl chloride with a DNA or RNA molecule as the capture molecule.

6. A method of manufacturing a biosensor comprising the steps of:
(i) providing a sensor base comprising an insulating substrate and at least one electrically conductive working electrode thereon,
(ii) providing reduced graphene oxide functionalized with difunctional amino compounds of different lengths in the form of sheet-like flakes of a size, i.e. maximum length, of 0.2 to 3.0 µm,
(iii) depositing the functionalized reduced graphene oxide provided in step (ii) onto the at least one working electrode by electrophoretic deposition; and
(iv) reacting a capture molecule with the product obtained in step (iii),
wherein the reduced graphene oxide is dispersed in a solvent under ultrasonic treatment between steps (ii) and (iii), and
wherein the faces of at least 50% of the graphene oxide flakes deviate from the plane of the working electrode face on which they are deposited.

7. The method according to claim 6, wherein a biosensor according to any one of claims 1 to 5 is obtained.

8. A method of manufacturing a biosensor comprising the steps of:
(i) providing a sensor base comprising an insulating substrate and at least one electrically conductive working electrode thereon,
(ii) providing reduced graphene oxide in the form of sheet-like flakes having a size, i.e. maximum length, of 0.2 to 3.0 µm,
(iii) functionalizing the reduced graphene oxide with difunctional amino compounds,
(iv) depositing the provided functionalized reduced graphene oxide onto the at least one working electrode by electrophoretic deposition; and
(v) reacting a capture molecule with the product obtained in step (iv),
wherein the reduced graphene oxide is dispersed in a solvent under ultrasonic treatment between steps (ii) and (iii) or between steps (iii) and (iv), and
wherein the faces of at least 50% of the graphene oxide flakes deviate from the plane of the working electrode face on which they are deposited.

9. The method of claim 8, wherein a biosensor according to any one of claims 1 to 5 is obtained.

10. A biochip carrying a biosensor according to any one of claims 1 to 5.

11. A method of detecting an analyte, which comprises contacting an analyte in a liquid medium with a biosensor according to any one of claims 1 to 5 or with a biochip according to claim 10, and subsequently measuring the change in an electrical property of the biosensor caused by the interaction of the analyte with the biosensor or biochip.

## Revendications

1. Biocapteur comprenant les éléments suivants :
(a) une base de capteur avec un substrat isolant et au moins une électrode de travail électro-conductrice située sur celui-ci,
(b) un oxyde de graphène réduit (rGO) appliqué sur au moins une des électrodes de travail sous la forme de flocons plats d'une grandeur, c'est-à-dire d'une longueur maximale, de 0,2 à 3,0 µm, les surfaces d'au moins 50 % des flocons d'oxyde de graphène se différenciant du plan de la surface des électrodes de travail sur laquelle ils sont appliqués,
(c) des éléments d'espacement (« Spacer ») de différentes longueurs, liés de manière covalente à l'oxyde de graphène réduit,
(d) respectivement une molécule de piégeage liée de manière covalente à l'élément d'espacement.

2. Biocapteur selon la revendication 1, dans lequel la molécule de piégeage est un fragment d'anticorps Fab ou un aptamère L.

3. Biocapteur selon la revendication 1 ou 2, dans lequel les éléments (b) à (d) sont appliqués sur une électrode de travail, où le biocapteur présente en outre une électrode de référence, ou sur des électrodes interdigitées qui peuvent être alimentées par un courant alternatif.

4. Biocapteur selon une des revendications précédentes, dans lequel les éléments d'espacement peuvent être des molécules de différentes longueurs de polyalkylène glycol et/ou des molécules de différentes longueurs de polyalkylène polyamine glycol aminées à l'extrémité.

5. Biocapteur selon une des revendications précédentes, dans lequel la liaison covalente entre l'élément d'espacement et la molécule de piégeage peut être obtenue par transformation d'un groupe carboxyle du fragment d'anticorps Fab servant de molécule de piégeage avec un groupe aminé de l'élément d'espacement ou par transformation d'un groupe aminé acidifié à l'aide d'un chlorure d'azidoacétyle avec une molécule d'ADN ou d'ARN servant de molécule de piégeage.

6. Procédé de fabrication d'un biocapteur comprenant les étapes suivantes :
(i) la préparation d'une base de capteur avec un substrat isolant et au moins une électrode de travail électro-conductrice située sur celui-ci,
(ii) la préparation d'un oxyde de graphène réduit, qui a été fonctionnalisé avec des liaisons amino difonctionnelles de différentes longueurs, sous la forme de flocons plats d'une grandeur, c'est-à-dire d'une longueur maximale, de 0,2 à 3,0 µm,
(iii) l'application de l'oxyde de graphène réduit fonctionnalisé préparé à l'étape (ii) sur l'au moins une électrode de travail par dépôt électrophorétique, et
(iv) la transformation d'une molécule de piégeage avec le produit obtenu à l'étape (iii),
dans lequel l'oxyde de graphène réduit est dispersé entre les étapes (ii) et (iii) dans un solvant sous un traitement par ultrasons, et
dans lequel les surfaces d'au moins 50 % des flocons d'oxyde de graphène se différencient du plan de la surface des électrodes de travail sur laquelle ils sont appliqués.

7. Procédé selon la revendication 6, dans lequel un biocapteur est obtenu selon une des revendications 1 à 5.

8. Procédé de fabrication d'un biocapteur comprenant les étapes suivantes :
(i) la préparation d'une base de capteur avec un substrat isolant et au moins une électrode de travail électro-conductrice située sur celui-ci,
(ii) la préparation d'un oxyde de graphène réduit sous la forme de flocons plats d'une grandeur, c'est-à-dire d'une longueur maximale, de 0,2 à 3,0 µm,
(iii) la fonctionnalisation de l'oxyde de graphène réduit avec des liaisons amino difonctionnelles,
(iv) l'application de l'oxyde de graphène réduit fonctionnalisé préparé sur l'au moins une électrode de travail à l'aide d'un dépôt électrophorétique et
(v) la transformation d'une molécule de piégeage avec le produit obtenu à l'étape (iv),
dans lequel l'oxyde de graphène réduit est dispersé entre les étapes (ii) et (iii) ou entre les étapes (iii) et (iv) dans un solvant sous un traitement par ultrasons, et
dans lequel les surfaces d'au moins 50 % des flocons d'oxyde de graphène se différencient du plan de la surface des électrodes de travail sur laquelle ils sont appliqués.

9. Procédé selon la revendication 8, dans lequel un biocapteur est obtenu selon une des revendications 1 à 5.

10. Biopuce qui porte un biocapteur selon une des revendications 1 à 5.

11. Procédé destiné à détecter un analyte, comprenant la mise en contact d'un analyte situé dans un milieu liquide avec un biocapteur selon une des revendications 1 à 5 ou avec une biopuce selon la revendication 10 et la mesure suivante du changement d'une propriété électrique du biocapteur, lequel est produit par l'interaction de l'analyte avec le biocapteur respectivement la biopuce.
